(19)

**Europäisches Patentamt**
**European Patent Office**
**Office européen des brevets**

(11) **EP 2 015 209 A2**

(12) **EUROPEAN PATENT APPLICATION**

(43) Date of publication:
**14.01.2009 Bulletin 2009/03**

(51) Int Cl.:
***G06F 19/00*** *(2006.01)*

(21) Application number: **08011637.9**

(22) Date of filing: **26.06.2008**

| | |
|---|---|
| (84) Designated Contracting States:<br>**AT BE BG CH CY CZ DE DK EE ES FI FR GB GR HR HU IE IS IT LI LT LU LV MC MT NL NO PL PT RO SE SI SK TR**<br>Designated Extension States:<br>**AL BA MK RS** | • **Chemical Computing Group Inc.**<br>**Montreal, QC H3A 2R7 (CA)**<br><br>(72) Inventors:<br>• **Arora, Nidhi**<br>**Cupertino, CA 95014 (US)**<br>• **Williams, Chris**<br>**Quebec, J7R 3T6 (CA)** |
| (30) Priority: **27.06.2007 US 823697** | |
| (71) Applicants:<br>• **F.HOFFMANN-LA ROCHE AG**<br>**4070 Basel (CH)** | (74) Representative: **Ritter, Thomas Kurt et al**<br>**Jones Day**<br>**Prinzregentenstraße 11**<br>**80538 München (DE)** |

(54) **Systems and methods for mapping binding site volumes in macromolecules**

(57)     Systems, methods, and apparatus for evaluating a binding site of a macromolecule are disclosed in which the binding site is identified and a binding site volume map of the binding site is constructed. The binding site volume map has a surface with a plurality of regions. Each respective region in the plurality of regions of the surface is classified based upon a first characteristic that is complementary to a second characteristic of a portion of the macromolecule that is nearest to the respective region, thereby creating an interaction map. Systems, methods, and apparatus for comparing macromolecules are also disclosed in which a binding site volume map is constructed for each of the macromolecules. Then, a composite binding site volume map that comprises a volumetric combination of each of the binding site volume maps is constructed. An interaction map can be constructed from the composite binding site volume map.

202
Identify the binding site of the macromolecule, where the binding site is lined by a plurality of residues and is preferably ligand free

204
Determine a binding site volume map of the first binding site, where the binding site volume map comprises a surface that has a plurality of regions (e.g. grid points)

206
Classify each respective region (*e.g.* respective grid point) in the plurality of regions of the surface of the binding site volume map based upon a characteristic that is complementary to a characteristic of a corresponding portion of the macromolecule (*e.g.* the residue in the macromolecule that is closest to the respective region), thereby creating an interaction map

208
Output the interaction map (*e.g.* to a user interface device, a monitor, a computer-readable storage medium, a computer-readable memory, or a local or remote computer system) or display the binding site volume map

Figure 2

EP 2 015 209 A2

**Description**

**CROSS REFERENCE TO RELATED APPLICATION**

This application claims priority to U.S. Patent Application No. 11/823,697 filed on June 27, 2007 which is hereby incorporated by reference herein in its entirety.

**BACKGROUND OF THE INVENTION**

**1. Field of Invention**

This invention relates to processes, apparatus, media and signals for mapping binding pocket volumes in macromolecules such as proteins and nucleic acids.

**2. Description of Related Art**

With the advent of several high-resolution macromolecular structures deposited in the Brookhaven Protein Databankl (PDB), structure-based drug design is more feasible. An example of the application of structure-based design techniques is the development of novel human immunodeficiency virus 1 (HIV-1) protease inhibitors. *See*, Rutenber et al., 1993, J. Biol. Chem. 268: 15343-15346; Ghosh et al., 1994, J. Med. Chem. 37: 2506-2508; and Lam et al., 1994, Science 263: 380-384. A prerequisite for the docking of small molecule ligands is the determination of the site where the ligand interacts with the protein. Such binding sites for small molecule ligands are pockets (alternatively and interchangeably named clefts, grooves, active sites) generally located near the surface of macromolecules such as proteins and nucleic acids. In the present application, the term "binding site" is used to refer to any such pocket of a macromolecule where a ligand binds or could potentially bind. The determination of binding sites is an important step toward the rational design and discovery of novel ligands. An in-depth analysis and classification of binding sites on the surfaces of the known macromolecular structures might also improve understanding of the processes involved in ligand binding and selectivity.

A manual definition of binding sites is laborious for several reasons. In most cases it is difficult to define exactly where a binding site ends and free space begins. Macromolecules may have surface depressions of various sizes and a manual inspection may fail to find all relevant binding sites but the largest one. A manual definition is also impractical when large sets of macromolecular structures must be processed (*e.g.,* for statistical studies of pocket characteristics). Several attempts to automate the identification of binding sites have been made over the years. See, for example, Levitt et al., 1992, J. Mol. Graphics 10: 229-234; Voorinthold et al., 1989, J. Mol. Graphics 7, 243 245; Delaney et al., 1992, J. Mol. Graphics 10: 174 177; Del Carpio et al., 1993, J. Mol. Graphics 11: 23 29, Kisljuk et al., 1994, J. Mol. Graphics 12: 305 307; Masuya et al., 1995, J. Mol. Graphics 13: 331 336; Laskowski et al., 1995, J. Mol. Graphics 13: 323 330; and Hendlich et al., 1998, J. Mol. Graphics 15: 359-363.

An important aspect of structure-based drug design is ligand specificity and ligand selectivity. It is desirable for a ligand to specifically bind to a binding site of the target macromolecule. Typically, specific binding of a ligand to the binding site of the target macromolecule alters a property of the macromolecule in a desirable way. For example, the macromolecule may be an enzyme and the enzymatic activity of the enzyme may be inhibited or enhanced upon specific binding of the ligand in the binding site of the enzyme. However, while a ligand may specifically bind to a binding site of a target macromolecule, it is desirable that the ligand also selectively bind to the binding site. For example, consider the case where the target macromolecule is a target kinase (*e.g.*, the P38A kinase) and the binding site is the active site of the target kinase where the natural ligand adenosine triphosphate binds. In the example, it is desirable to inhibit the target kinase (*e.g.*, inhibit the enzymatic activity of P38A). Therefore, a ligand that specifically binds the active site (binding site) of the target kinase is desirable. However, if this ligand also specifically binds to the active site of many other types of kinases other than the target kinase (*e.g.*, the kinase CDK2, *etc.*), the ligand is likely to be toxic because it would interfere with a large number of molecular pathways in the cell that are regulated by these other kinases. A ligand that specifically binds many different macromolecules in this way does not have the desired selectively. On the other hand, if the ligand only specifically binds to the binding site of the target kinase and not other kinases, the ligand has the desirable selectivity.

Existing programs for identifying and analyzing binding sites of macromolecules have considerable utility. However, one deficiency with such known programs is that they do not provide convenient ways to analyze ligand selectivity. Furthermore, improvement is needed in analyzing binding sites of macromolecules in order to visualize the properties (*e.g.*, acidic, basic, polar, nonpolar, *etc.*) that a potential ligand should have in order to be complementary to the properties exhibited by the binding site of the macromolecule. Thus, that are needed in the art are improved systems and methods for identifying and analyzing pockets of macromolecules.

## SUMMARY OF THE INVENTION

The present invention provides processes, apparatus, media and signals that have many useful applications including, but not limited to, identifying vectors for ligand design, quick generation of ligand selectivity hypotheses, and the identification of common volumes in the binding pockets of different macromolecules for addressing multiple targets. In one embodiment, quick identification of macromolecule binding sites using alpha spheres is followed by mapping of the binding site volume graphically as well as numerically. The graphical volume map defines the volume available to a potential ligand in the macromolecule binding site. This volume map can be enhanced through, for example, color coding to form an interaction map that defines the expected interaction types (*e.g.* hydrophobic, electrostatic, *etc.*) that the ligand atoms need to adopt in order to achieve complementarity with the binding site of the macromolecule. Intersection of the volume maps of related macromolecules allows for the identification of unique regions that can be exploited to achieve selectivity.

In particular, one aspect of the invention provides improved processes, apparatus, media and signals for evaluating a binding site of a macromolecule, where the binding site is lined by a plurality of residues and where the binding site is preferably ligand free. First, the binding site of the macromolecule is identified and then a binding site volume map of the binding site is determined. The binding site volume map comprises a surface that has a plurality of regions. An interaction map is created by classifying each respective region in a plurality of regions of the surface of the binding site volume map based upon a first characteristic that is complementary to a second characteristic of a portion of the macromolecule that is nearest to the respective region, thereby creating an interaction map. The interaction map is outputted to a user interface device, a monitor, a computer-readable storage medium, a computer-readable memory, or a local or remote computer system. Alternatively, the binding site volume map is displayed.

In some embodiments, the macromolecule is a protein while in other embodiments the macromolecule is a nucleic acid. In some embodiments, the identifying step comprises searching a model of the macromolecule for the binding site using an energetic function, a grid based algorithm, a geometric algorithm, a classification method, or a direct method. Of course, a macromolecule may have more than one binding site, each of which can be analyzed individually using the systems and methods of the present invention. In some embodiments, the identifying step comprises searching a model of the macromolecule for the binding site using the alpha spheres, which is one type of geometric method for identifying binding sites.

In some embodiments, the second characteristic of a respective portion of the macromolecule that is nearest to a respective region in the plurality of regions of the surface of the binding site volume map is a hydrophobicity of one or more atoms in the residue in the plurality of residues that is nearest the respective portion of the macromolecule. In some embodiments, the second characteristic of a respective portion of the macromolecule that is nearest to a respective region in the plurality of regions of the surface of the binding site volume map is an amount of solvent exposure of one or more atoms in the residue in the plurality of residues that is nearest the respective portion of the macromolecule. In some embodiments, the second characteristic of a respective portion of the macromolecule that is nearest to a respective region in the plurality of regions of the surface of the binding site volume map is a classification of each of one or more atoms in a residue that is nearest the respective portion of the macromolecule as acidic, basic, hydrophobic, or solvent exposed. In some embodiments, the classification of a respective region in the plurality of regions of the surface of the binding site (pocket) volume map comprises coloring the respective region based upon the characteristic of the one or more atoms that are nearest the respective region.

In some embodiments, the identifying step comprises the following steps for generation of alpha spheres. In step (i) the Voronoi region of each atom of the macromolecule is determined, thereby determining a plurality of Voronoi regions. In step (ii) each Voronoi vertice in the plurality of Voronoi regions is determined, thereby determining a plurality of Voronoi vertices. In step (iii) an alpha sphere is assigned to each Voronoi vertice in the plurality of Voronoi vertices, thereby determining a first plurality of alpha spheres. In optional step (iv) alpha spheres in the first plurality of alpha spheres that have a diameter below a first threshold and alpha spheres in the plurality of alpha spheres that have a diameter above a second threshold are eliminated, thereby identifying a filtered second plurality of alpha spheres. In step (v) each respective sphere in the second plurality of spheres is classified as a hydrophilic sphere or a hydrophobic sphere based on an ability of the respective sphere to form a hydrogen bond with an atom of the macromolecule. In step (vi) the alpha spheres in the second plurality of alpha spheres are clustered thereby forming a plurality of candidate binding sites, where each respective candidate binding site in the plurality of candidate binding sites comprises three or more alpha spheres in the second plurality of spheres, and where at least one alpha sphere in the three or more alpha spheres is hydrophobic. In step (vii) each respective candidate binding site in the plurality of candidate binding sites is ranked by a number of hydrophobic contacts made by the respective candidate binding site to the macromolecule, thereby forming a ranked set of candidate binding sites. In step (viii), the binding site is determined from the ranked set of candidate binding sites. In some embodiments, the method further comprises the step of eliminating near duplicate alpha spheres in the second plurality of alpha spheres prior to the clustering step (vi). In some embodiments, the determining step (viii) comprises receiving a selection of a candidate binding site in the plurality of candidate binding sites from a user, where the candidate binding site selected by the user is deemed to be the binding site. In some embodiments, the determining step (viii) comprises deeming the candidate binding site in the ranked set of candidate binding sites that has a maximum number of hydrophobic contacts with the macromolecule to be the binding site. In some embodiments, the clustering step (vi) comprises single-linkage clustering of the plurality of second plurality of alpha spheres.

In some embodiments, the binding site volume map of the binding site is determined by placing a grid on the alpha spheres in the binding site, where the grid comprises a plurality of grid points. The binding site volume map is identified as all grid points in the grid that satisfy the criteria of (a) falling inside the alpha spheres of the binding site map and (b) being within a predetermined distance of at least one atom of the macromolecule. In some embodiments, the predetermined distance is a distance between 1.2 Angstroms and 2.0 Angstroms. In some embodiments, the predetermined distance is between 1.35 Angstroms and 1.85 Angstroms. In some embodiments, each respective region in a plurality of regions of the surface of the binding site volume map is a one of the above-identified grid points and the second characteristic of a portion of the macromolecule that is nearest to the respective grid point are one or more molecular properties of the residue in the amino acid that is closest to the grid point (*e.g.*, the molecular properties of the amino acids set forth in Table 1, below).

Another aspect of the invention provides a method of comparing a first binding site of a first macromolecule to a second binding site of a second macromolecule, where the first binding site and the second binding site are preferably ligand free. The method comprises identifying the first binding site of the first macromolecule as well as the second binding site of the second macromolecule. The method further comprises determining a first binding site volume map based upon the first binding site as well as determining a second binding site volume map based upon the second binding site. A third binding site volume map that comprises a volumetric combination of the first binding site volume map and the second binding site volume map is then generated. The third binding site volume map is outputted to a user interface device, a monitor, a computer-readable storage medium, a computer-readable memory, or a local or remote computer system, or the third binding site volume map is displayed. In some embodiments, the first macromolecule is a first protein and the second macromolecule is a second protein. In some embodiments, the first macromolecule is a first nucleic acid and the second macromolecule is a second nucleic acid.

In some embodiments, the identification of the first binding site of the first macromolecule comprises searching a first model of the first macromolecule for the first binding site using an energetic function, a grid based algorithm, a geometric algorithm, a classification method, or a direct method. In some embodiments, the identification of the first binding site of the first macromolecule comprises searching a first model of the first macromolecule for the first binding site using alpha spheres, which is one form of geometric method. In some embodiments, the identification of the second binding site of the second macromolecule comprises searching a second model of the second macromolecule for the second binding site using an energetic function, a grid based algorithm, a geometric algorithm, a classification method, or a direct method. In some embodiments, the identification of the second binding site of the second macromolecule comprises searching a second model of the second macromolecule for the second binding site using the alpha spheres.

In some embodiments the volumetric combination is a union of the first binding site volume map and the second binding site volume map. In some embodiments, the volumetric combination is a difference of the first binding site volume map and the second binding site volume map. In some embodiments, the volumetric combination is an intersection of the first binding site volume map and the second binding site volume map. In some embodiments, the volumetric combination is an average of the first binding site volume map and the second binding site volume map. In some embodiments, the volumetric combination is a uniqueness function and the third binding site volume map consists of (i) those portions of the first binding site volume map that are not shared by the second binding site volume map and (ii) those portions of the second binding site volume map that are not shared by the first binding site volume map.

In some embodiments the identification of the first binding site comprises the following steps. In step (i) the Voronoi region of each atom of the first macromolecule is determined, thereby determining a plurality of Voronoi regions. In step (ii) each Voronoi vertice in the plurality of Voronoi regions is determined, thereby determining a plurality of Voronoi vertices. In step (iii) an alpha sphere is assigned to each Voronoi vertice in the plurality of Voronoi vertices thereby determining a first plurality of alpha spheres. In optional step (iv) alpha spheres in the first plurality of alpha spheres that have a diameter below a first threshold and alpha spheres in the plurality of alpha spheres that have a diameter above a second threshold are eliminated, thereby identifying a filtered second plurality of alpha spheres. In step (v) each respective sphere in the second plurality of spheres is classified as a hydrophilic sphere or a hydrophobic sphere based on an ability of the respective sphere to form a hydrogen bond with an atom of the macromolecule. In step (vi) the alpha spheres in the second plurality of alpha spheres are clustered thereby forming a plurality of candidate binding sites, where each respective candidate binding site in the plurality of candidate binding sites comprises three or more alpha spheres in the second plurality of alpha spheres, and where at least one alpha sphere in the three or more alpha spheres is hydrophobic. In step (vii) each respective candidate binding site in the plurality of candidate binding sites is ranked by a number of hydrophobic contacts made by the respective candidate binding site to the macromolecule, thereby forming a ranked set of candidate binding sites. In step (viii), a determination of the binding site from the ranked set of candidate binding sites is made. In some embodiments, near duplicate alpha spheres in the second plurality of alpha spheres prior to the clustering step (vi). In some embodiment, the determining step (viii) comprises receiving a selection of a candidate binding site in the plurality of candidate binding sites from a user, where the candidate binding site selected by the user is deemed to be the binding site. In some embodiments, the determining step (viii) comprises deeming the candidate binding site in the ranked set of candidate binding sites that has a maximum number of hydrophobic contacts with the macromolecule to be the binding site. In some embodiments, the clustering step (vi) comprises single-linkage clustering of the plurality of second plurality of alpha spheres.

In some embodiments, the determination of the first binding site volume map comprises placing a grid on the alpha spheres in the first binding site, where the grid comprises a plurality of grid points. Then, the first binding site volume map is identified as all grid points in the grid that satisfying the criteria of (i) falling inside the alpha spheres of the binding site map and (ii) are being a predetermined distance of at least one atom of the first macromolecule (meaning that the grid points must satisfy both criterion (i) and (ii). In some embodiments, the predetermined distance is a distance between 1.2 Angstroms and 2.0 Angstroms. In some embodiments, the predetermined distance is between 1.35 Angstroms and 1.85 Angstroms. In some embodiments, an interaction map is generated from the third binding site volume map (the composite binding site volume map) in which each grid point in the binding site volume map is assigned a first characteristic that is complementary to a second characteristic of a portion of the macromolecule that is nearest to the respective grid point. In some embodiments, the portion of the macromolecule that is nearest to the respective grid point is a residue and the second characteristic is one or more molecular properties of the residue (*e.g.*, the molecular properties of the amino acids set forth in Table 1, below).

Another aspect of the present invention provides a computer-readable medium storing a computer program product, executable by a computer, to evaluate a binding site of a macromolecule. The computer program product comprises instructions for identifying the binding site of the macromolecule, where the binding site is lined by a plurality of residues and where the binding site is optionally, but preferably, ligand free. The computer program product further comprises instructions for determining a binding site volume map of the first binding site, where the binding site volume map comprises a surface that has a plurality of regions. The computer program product further comprises instructions for classifying each respective region in the plurality of regions of the surface of the binding site volume map (*e.g.*, each grid point in the binding site volume map) based upon a first characteristic that is complementary to a second characteristic of a portion of the macromolecule that is nearest to the respective region, thereby creating an interaction map. In some embodiments, the computer program product further comprises instructions for outputting the interaction map to a user interface device, a monitor, a computer-readable storage medium, a computer-readable memory, or a local or remote computer system. Alternatively or additionally, in some embodiments, the computer program product comprises instructions for displaying the binding site volume map.

Another aspect of the invention comprises an apparatus for evaluating a binding site of a macromolecule, the apparatus comprising a processor and a memory, coupled to the processor. The memory stores a module comprising instructions for identifying the binding site of the macromolecule, where the binding site is lined by a plurality of residues and where the binding site is optionally, but preferably, ligand free. The module further comprises instructions for determining a binding site volume map of the first binding site, where the binding site volume map comprises a surface that has a plurality of regions (*e.g.*, grid points). The module further comprises instructions for classifying each respective region in the plurality of regions of the surface of the binding site volume map based upon a first characteristic that is complementary to a second characteristic of a portion of the macromolecule that is nearest to the respective region (*e.g.*, the nearest residue on the macromolecule), thereby creating an interaction map. In some optional embodiments, the module further comprises instructions for outputting the interaction map to a user interface device, a monitor, a computer-readable storage medium, a computer-readable memory, or a local or remote computer system. In some optional embodiments, the module comprises instructions for displaying the binding site volume map.

Still another aspect of the present invention comprises a computer-readable medium storing a computer program product, executable by a computer, to compare a first binding site of a first macromolecule to a second binding site of a second macromolecule, where the first binding site and the second binding site are optionally, but preferably, ligand free. The computer program product comprises instructions for identifying the first binding site of the first macromolecule as well as the second binding site of the second macromolecule. The computer program product further comprises instructions for determining a first binding site volume map based upon the first binding site as well as a second binding site volume map based upon the second binding site. The computer program product further comprises instructions for generating a third binding site volume map that is a volumetric combination of the first binding site volume map and the second binding site volume map. The computer program product optionally comprises instructions for outputting the third binding site volume map to a user interface device, a monitor, a computer-readable storage medium, a computer-readable memory, or a local or remote computer system. The computer program product optionally comprises instructions for displaying the third binding site volume map.

Another aspect of the present invention provides an apparatus for comparing a first binding site of a first macromolecule to a second binding site of a second macromolecule, where the first and second bindings site are optionally, but preferably, ligand free. The apparatus comprises a processor and a memory, coupled to the processor. The memory stores a module comprising instructions for identifying the first binding site of the first macromolecule as well as the second binding site of the second macromolecule. The memory further stores instructions for determining a first binding site volume map based upon the first binding site as well as a second binding site volume map based upon the second binding site. The memory further stores instructions for generating a third binding site volume map that is a volumetric combination of the first binding site volume map and the second binding site volume map. The memory optionally further stores instructions for outputting the third binding site volume map to a user interface device, a monitor, a computer-readable storage medium, a computer-readable memory, or a local or remote computer system. The memory further optionally stores instructions for displaying the third binding site volume map.

## BRIEF DESCRIPTION OF THE DRAWINGS

Figure 1 illustrates an apparatus for mapping binding site volumes in macromolecules, such as proteins and nucleic acids, as well as comparing binding sites of macromolecules in accordance with an aspect of the disclosure.

Figure 2 illustrates a method for mapping binding site volumes in macromolecules in accordance with an embodiment of the present invention.

Figure 3 illustrate the Voronoi decomposition of a set of zero-radii atoms in two dimensions, where the atoms are shown as full larger circles, and the boundaries of the Voronoi regions are shown as lines.

Figure 4 illustrates a method for comparing binding sites of macromolecule in accordance with an embodiment of the present invention.

Figure 5A illustrates a composite binding site volume between the macromolecule P38A and JNK that is an intersection volume in accordance with an embodiment of the present invention.

Figure 5B illustrates the inhibitor R1487 superimposed on a binding site volume map that is the intersection of the P38AA and JNK3 binding site volume maps in accordance with an embodiment of the present invention.

Figure 5C illustrates the molecular structure of R1487

Figure 6A illustrates the inhibitor RO7125 superimposed onto a volume map that is the volumetric combination of the binding site volume map of the P38AA kinase and the binding site volume map of the JNK3 kinase in which only those regions unique to the P38AA binding site volume map are kept in the volumetric combination. In the present invention it is possible to color code the surface of the unique volume based on attributes of the P38AA volume map.

Figure 6B illustrates the molecular structure of RO7125.

Figure 7A illustrates the inhibitor RO9552 superimposed onto a volume map that is the volumetric combination of the binding site volume map of the P38AA kinase and the binding site volume map of the JNK kinase in which only those regions unique to the P38AA binding site volume map are kept in the volumetric combination.

Figure 7B illustrates the molecular structure of RO9552.

Figure 8 illustrates the inhibitor R06257 bound in the binding site of the P38AA kinase with an interaction map of the binding site drawn, in accordance with an embodiment of the present invention.

## DETAILED DESCRIPTION

Residues lining the binding site within a macromolecule fold define a unique volume of the ligand binding site. Mapping the protein binding site alone provides a "macromolecule perspective" of expected shape and interactions of a potential inhibitor. Variations in binding sites of related molecules can then be explored to identify regions for targeting selectivity. Accordingly, the present invention provides systems and methods for evaluating a binding site of a macromolecule. The systems and methods have advantageous application in the field of ligand design. For instance, the systems and methods of the present invention can be used to generate interaction maps that allow for the visualization of available space and expected ligand-macromolecule intermolecular interactions in the binding site. Furthermore, the systems and methods provide novel abilities to analyze ligand cross-reactivity. This is accomplished by performing set operations (intersection, unique) on the binding site volume map of two or more macromolecules. Such set operations allow for the visualization of commonality or differences between binding sites of two or more macromolecules.

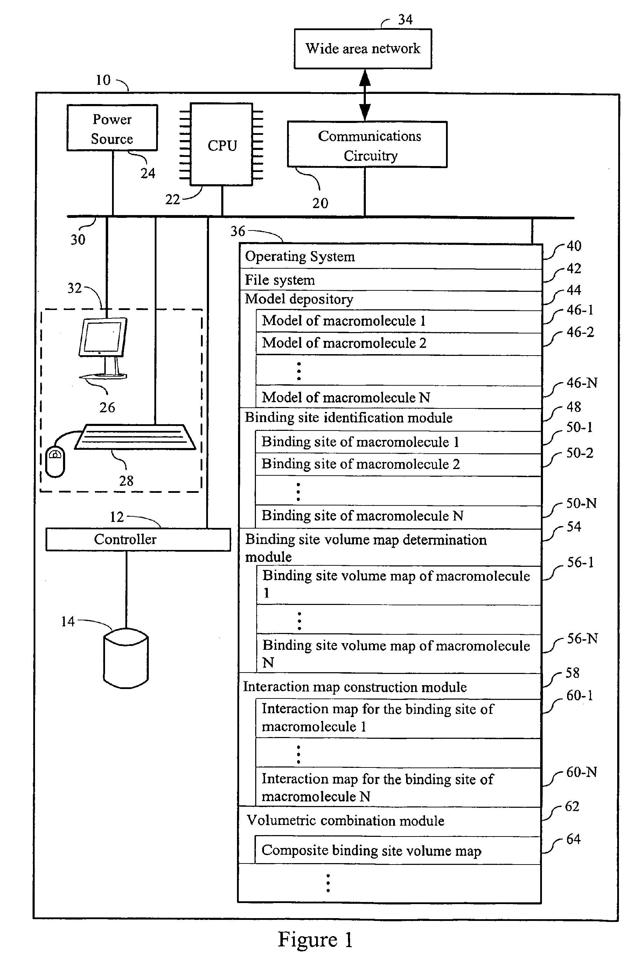

*Exemplary Computer Systems.* Figure 1 details an exemplary system for performing any of the methods disclosed herein. The system is preferably a computer system 10 having:

- a central processing unit 22;
- a main non-volatile storage unit 14, for example, a hard disk drive, for storing software and data, the storage unit 14 controlled by storage controller 12;
- a system memory 36, preferably high speed random-access memory (RAM), for storing system control programs, data, and application programs, comprising programs and data loaded from non-volatile storage unit 14; system memory 36 may also include read-only memory (ROM);
- a user interface 32, comprising one or more input devices (*e.g.*, keyboard 28) and a display 26 or other output device;
- a network interface card or other communications circuitry 20 for connecting to any wired or wireless communication network 34 (*e.g.*, a wide area network such as the Internet);
- an internal bus 30 for interconnecting the aforementioned elements of the system; and
- a power source 24 to power the aforementioned elements.

Operation of the computer 10 is controlled primarily by an operating system 40, which is executed by a central processing unit 22. Operating system 40 can be stored in system memory 36. In addition to an operating system 40, in a typical implementation, a system memory 36 includes:

- a file system 42 for controlling access to the various files and data structures used by the disclosed systems and methods;
- a model depository 44 comprising a plurality of models of macromolecules 46 (*e.g.*, determined by X-ray crystallography, nuclear magnetic resonance, homology modeling, or other spectroscopic or modeling methods), in which each model comprises a plurality of atomic coordinates for a macromolecule;
- a binding site identification module 48 for determining one or more candidate binding sites of a macromolecule and optionally identifying one of the candidate binding sites as the binding site 50 of the macromolecule;
- a binding site volume map determination module 54 for determining a binding site volume map 56 of a binding site 50 of a model of a macromolecule 46;
- an interaction map construction module 58 for classifying each respective region in a plurality of regions of the surface of the binding site volume map 56 of a model of a macromolecule 46 based upon a characteristic of one or more residues in the plurality of residues in the macromolecule that are nearest to the respective region, thereby creating an interaction map 60 for the binding site 50 of the macromolecule 46; and
- a volumetric combination module 62 for generating a composite binding site volume map 64 that is a volumetric combination of at least a first binding site volume map 56 and a second binding site volume map 56.

As illustrated in Figure 1, computer 10 comprises software program modules and data structures. The data structures stored in computer 10 include model depository 44, models 46, binding sites 50, binding site volume maps 56, interaction maps 60, and composite binding site volume maps 64. Each of these data structures can comprise any form of data structure including, but not limited to, an ASCII or binary file, an Excel spreadsheet, a relational database (SQL), or an on-line analytical processing (OLAP) database (MDX and/or variants thereof). Further, when in the form of an ASCII or binary file, the data structures can be in any file format conventionally known in the art or otherwise.

In some embodiments, each of the aforementioned data structures is a single data structure. In other embodiments, such data structures in fact comprise a plurality of data structures (*e.g.*, databases, files, archives) that may or may not all be hosted by the same computer 10. For example, in some embodiments, model depository 44 and the models 46 in the depository are stored either on computer 10 and/or on one or more computers that are addressable by computer 10 across wide area network or Internet 34. Thus, in some embodiments, any of the aforementioned data structures can be (i) stored on computer 10, (ii) stored on a combination of computer 10 and other computers (not illustrated in Figure 1) that are addressable by computer 10 across, for example, wide area network 34, or (iii) are remotely stored in their entirety on one or more other computers (not illustrated in Figure 1) that are addressable by computer 10 across, for example, wide area network or Internet 34.

As in the case of the data structures, it will be appreciated that many of the modules illustrated in Figure 1 can also be located on one or more remote computers. For example, in some embodiments the disclosed methods are implemented as web service services. In such embodiments, binding pocket identification module 48, binding site volume map determination module 54, interaction map construction module 58 and/or volumetric combination module 62 can reside on a client computer that is in communication with computer 10 via network 34. In some embodiments, for example, any combination of binding pocket identification module 48, binding site volume map determination module 54, interaction map construction module 58 and volumetric combination module 62 can be an interactive web page.

In view of the foregoing, any arrangement of the data structures and software modules illustrated in Figure 1 on one or more computers is within the scope of the present invention so long as these data structures and software modules are addressable with respect to each other across network 34 or by other electronic means. Thus, the present invention fully encompasses a broad array of computer systems.

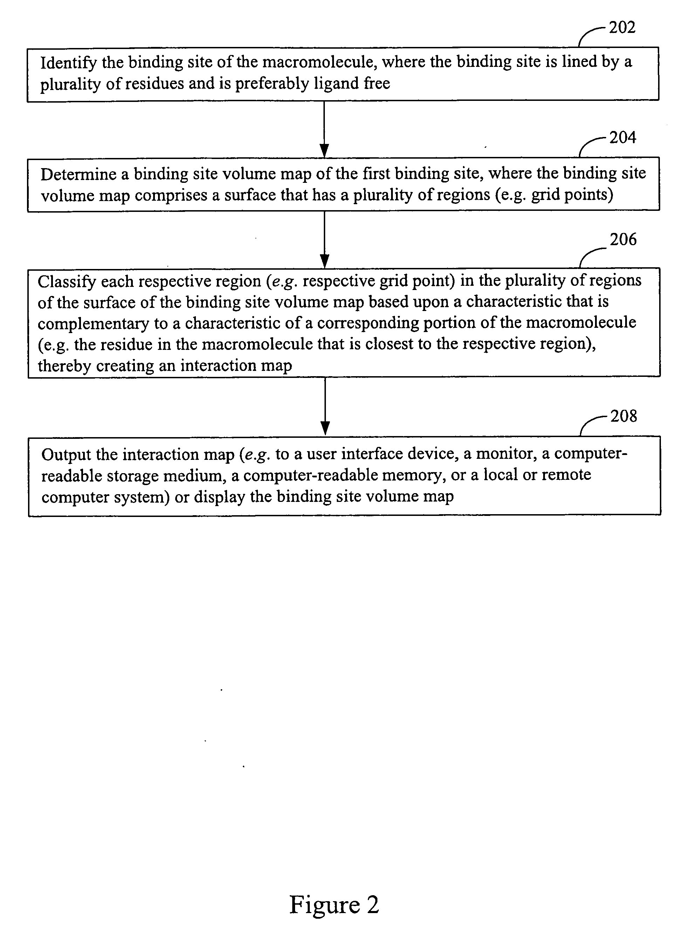

*Evaluating a macromolecule binding site.* Exemplary computer systems have now been disclosed. Turning to Figure 2, a method of evaluating a binding site 50 of a macromolecule 46, such as a protein or nucleic acid, is disclosed.

*Step 202.* In the method, at step 202 the binding site 50 of the macromolecule 46 is identified. As used herein the term "macromolecule" and "a model for a macromolecule" are used interchangeably. The binding site is lined by a plurality of residues and typically is ligand free during the binding site identification phase. The macromolecule 46 can be, for example, a protein, a peptide, a protein-protein complex, a protein-nucleic acid complex, or a nucleic acid. A binding site of a macromolecule 46 is a site on the surface of the macromolecule that specifically associates with a substrate or ligand in order to accomplish a biological function. For example, in the case of a protein, this biological function may be a protein activity such as, for example, an enzymatic activity. Identifying the plurality of residues in the protein that line the binding site facilitates rational drug design, where inhibitors are designed to interact with such residues. It is expected that inhibitors that tightly interact with residues that line a binding site (inhibitor specifically binds to the macromolecule binding site)) will inhibit some characteristics of the protein, including, but not limited to, enzymatic activity associated with the protein. Thus, predicting the binding sites of proteins and other macromolecules represents an import challenge in computational molecular biology. See, Irving et al., 2001, Proteins 42, 378-382, which is hereby incorporated by reference herein.

In some embodiments, binding site identification module 48 is used to identify the binding site 50 of the macromolecule 46. Any cavity finder protocol may be used to find the binding site on the macromolecule in step 202. Thus, any such cavity finder protocol may serve as binding site identification module 48. Exemplary protocols for finding binding sites are set forth in the section entitled "Exemplary Methods for Identifying a Binding Site in a Macromolecule," below.

In one embodiment, the binding site of the macromolecule is identified using alpha spheres, which is one form of geometric (non-energy based) method for finding binding pockets. Location of binding sites in protein structures using alpha spheres has been described by Labute and Santavy of the Chemical Computing Group (Montreal, Quebec Canada H3A 2R7) and is implemented in the Chemical Computing Group program Site Finder. Alpha spheres (See also Edelsbrunner et al., 1995, Proc. of the 28th Annual Hawaii Intl. Conf. on Systems Science 256-264, which is hereby incorporated by reference herein) are spheres of varying radii that must touch four atoms of the receptor on its boundary and do not contain any internal atoms.

Note that a sphere that contacts four atoms is unique: there is no other sphere that contacts the same four atoms. A special case of an alpha sphere is an empty half-space (a half of space separated by a plane from the other half) that contacts three atoms. Such a half-space is considered herein to be an alpha sphere of infinite radius with infinity as the fourth contacting point.

Alpha spheres are identified using the following process. First, the Voronoi region of each atom of the protein is determined. This is referred to as a Voronoi decomposition of space. In three dimensions, the point at which four Voronoi regions intersect is referred to as a Voronoi vertex. These Voronoi vertices form the centers of alpha spheres. Thus, the problem of finding alpha spheres can be transformed into the problem of finding the Voronoi decomposition of space, given the set of atoms that constitute the macromolecule. The Molecular Operating Environment (MOE) uses a fast method that allows collection of these alpha spheres into Voronoi vertices in seconds instead of minutes per protein. In the MOE approach as implemented in Site Finder, a "perpendicular" distance definition is used, which defines the distance to a sphere representing an atom as the distance to the intersection of the sphere representing the atom with a tangent to it:

$$\text{distance}^2(\text{point}_A, \text{sphere}_B) = \text{distance}_2(\text{point}_A, \text{center}_B) - \text{radius}^2_B$$

This leads to computationally tractable Voronoi regions with linear boundaries, while not significantly affecting the positions of the resulting alpha spheres.

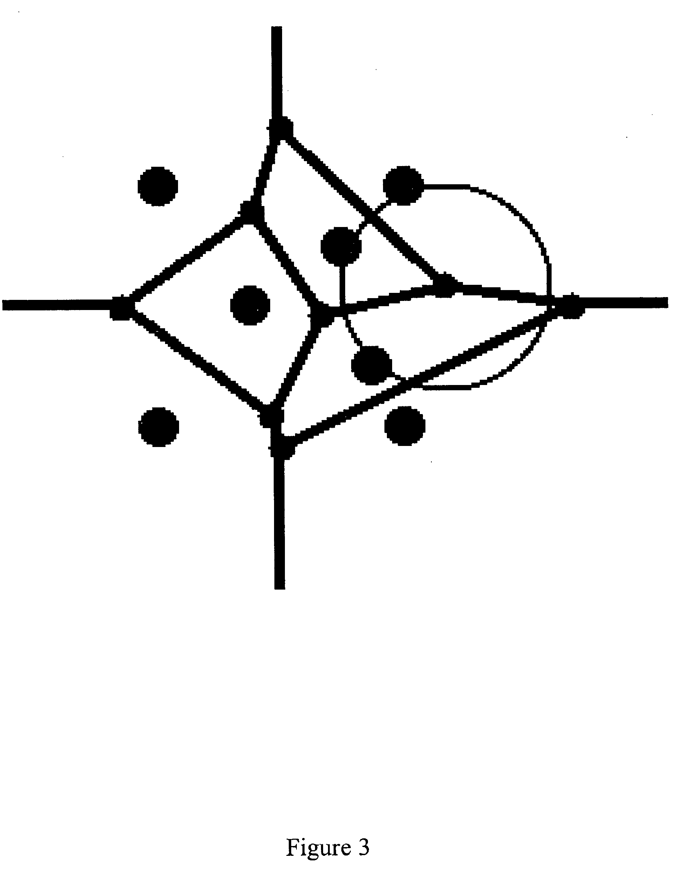

Figure 3 shows the Voronoi decomposition of a set of zero-radii atoms in two dimensions. The atoms are shown as full larger circles. The boundaries of the Voronoi regions are shown as lines. The Voronoi vertices are the vertices of these lines. Voronoi vertices in two-dimensions are intersections of just three, not four, Voronoi regions. The large thin circle shows one of the alpha spheres. Preferably, the alpha spheres are filtered based on size. Smaller spheres representing inaccessible tight binding regions, as well as very large spheres representing solvent exposed regions are eliminated, while retaining the intermediate spheres that represent regions that can be occupied by a ligand. In the MOE Site Finder program, the alpha spheres are generated as spheres inside tetrahedra. The center of the tetrahedron is the center of the alpha sphere, and lines drawn from the center of the tetrahedron are the "axes" of the alpha sphere. Alpha spheres are kept that satisfy each of the following criteria: (i) the sphere radius is less than 5 Å, no tetrahedron axes exceeds 40 Å, and only one tetrahedron axes exceeds 6 Å. In some embodiments, an additional criterion is imposed in order to ensure that alpha pockets within deep pockets are preserved. Accordingly, a buffer that increases the alpha sphere radii by one angstrom is added only to alpha spheres with a radius of 1.1 Angstrom or less in order to preserve the tight sub-pockets without overly increasing the pocket size in the region of large alpha spheres.

Each alpha sphere is classified as hydrophilic or hydrophobic. An alpha sphere that is positioned such that it can form a hydrogen bond with an atom of the macromolecule is classified as hydrophilic. Thus, an alpha sphere that is positioned within hydrogen bonding distance of a hydrogen bond donor (*e.g.*, a hydrogen attached at an electronegative atom such as oxygen or nitrogen) or a hydrogen bond acceptor (*e.g.*, fluorine, oxygen or nitrogen) is classified as hydrophilic. An alpha sphere that is positioned such that is cannot form a hydrogen bond with an atom of the macromolecule is classified as hydrophobic. In some embodiments, the probe radii are set at 1.3 Angstroms for hydrophilic alpha spheres and 1.7 Angstroms for hydrophobic alpha spheres.

Next, near duplicate alpha spheres are eliminated. For example, alpha spheres whose radii overlap each other are eliminated. Furthermore, in some embodiments, hydrophilic spheres not near a hydrophobic sphere are eliminated because such sites generally correspond to water sites. The macromolecular binding site is then identified by clustering alpha spheres using, for example, a single-linkage clustering algorithm. Each candidate binding site consists of three or more spheres at least one of which is hydrophobic. In some embodiments, the candidate binding sites are ranked by number of hydrophobic contacts made to the receptor, the highest one having the maximum hydrophobic contacts. In some embodiments, multiple candidate binding sites are displayed and made available for selection. Candidate binding sites can also be joined by a user based on an understanding of the macromolecule binding pocket. Ultimately, in typical embodiments, a single candidate binding site is selected for analysis although the macromolecule may have several candidate binding sites.

*Step 204. Generate binding site volume map.* In step 204, a binding site volume map 56 of the macromolecule 46 is determined. In some embodiments, the binding site volume map 56 is determined by binding site volume map determination module 54. In some embodiments, volume map determination module 54 comprises a custom Svl script for use with the Molecular Operating Environment (MOE) to calculate the binding site volume map 56.

In some embodiments, the binding site volume map 56 is determined by fitting a three-dimensional box on the binding site 50 identified by binding site identification module 48. For instance, consider the case where alpha spheres were used to identify a binding site 50 of a macromolecule. A three-dimensional box is fitted over the alpha spheres. Each dimension of the three-dimensional box is bounded by the alpha spheres or selected dummy atoms. For example, in some embodiments, the minimum x-dimension of the three-dimensional box is defined by a minimum value on the x-dimension that is occupied by any portion of any of the alpha spheres that constitute the binding site, the maximum x-dimension of the three-dimensional box is defined by a maximum value on the x-dimension that is occupied by any portion of any of the alpha spheres that constitute the binding site, the minimum *y*-dimension of the three-dimensional box is defined by a minimum value on the y-dimension that is occupied by any portion of any of the alpha spheres that constitute the binding site, the maximum y-dimension of the three-dimensional box is defined by a maximum value on the y-dimension that is occupied by any portion of any of the alpha spheres that constitute the binding site, the minimum z-dimension of the three-dimensional box is defined by a minimum value on the z-dimension that is occupied by any portion of any of the alpha spheres that constitute the binding site, and the maximum z-dimension of the three-dimensional box is defined by a maximum value on the z-dimension that is occupied by any portion of any of the alpha spheres that constitute the binding site. It will be appreciated that the three-dimensional grid box is not required. It merely provides a computationally convenient method for ensuring that grid points within each of the alpha spheres are spaced in a uniform manner. Other methods for ensuring uniform spacing of all the grid points through the plurality of alpha spheres that constitute the binding site 50 may be used.

In some embodiments, the grid points that are used to fill the alpha spheres are on a 1.0Å spacing. Uniform spacings smaller than or larger than 1.0Å could alternatively be used. For example, the grid points could be uniformly spaces at 0.5Å spacings in each of the three Cartesian dimensions. In another example, the grid points could be uniformly spaces at 1.5Å in each of the three Cartesian dimensions. Other possible uniform spacing values that could be used to space the grid points in three dimensions include, but are not limited to 0.1Å, 0.2Å, 0.3Å, 0.4Å, 0.5Å, 0.6Å, 0.7Å, 0.8Å, 0.9Å, 1.0Å, 1.1Å, 1.2Å, 1.3Å, 1.4Å, and 1.5Å, or any value between 0.01Å, and 3.0Å.

Once the three-dimensional box has been fitted over the grid points, all grid points that fall inside the alpha spheres and that are also within a predetermined distance of any atom of the macromolecule 46 are retained. In some embodiments this predetermined distance is between 1.2 Angstroms and 2.0 Angstroms. In some embodiments this predetermined distance is between 1.35 Angstroms and 1.85 Angstroms. In some embodiments, this predetermined distance is 1.4Å. In some embodiments, this predetermined distance is 1.8Å.

The grid points that satisfy both these criteria form the binding site volume map 50 of the macromolecule 46. In some embodiments, these grid points are used to generate a binding site volume map. In some embodiments, dummy atoms are placed on each of the retained grid points and a three-dimensional surface (*e.g.*, a Connolly surface, a Van der Waals surface or solvent accessible surface) is drawn over the grid points. For computation of such surfaces see, for example, Connolly, 1983, J. Appl. Cryst. 16: 548-558, which is hereby incorporated by reference herein. In some embodiments, the surface of the binding site volume map is computed and displayed as a MOE Isosurface. In some embodiments, the surface of the binding site volume map is computed and displayed as a Pymol (DeLano, "The PyMOL Molecular Graphics System," DeLano Scientific LLC, Palo Alto, California) Gaussian surface. Both the grid points that fill the binding pocket and any three-dimensional surface drawn over such grid points may be referred to herein, interchangeably, as the binding volume map 56. Furthermore, any other three-dimensional representation of the binding site 50 is deemed to be a binding site volume map 56. In one specific embodiment, the binding volume map 56 comprises grid points that can be read into a program such as PyMOL (DeLano, 2007, DeLano Scientific LLC, Palo Alto, California) as a set of points on a regular grid as output from the Molecular Operating Environment. In this specific embodiment, the points are then converted to a volumetric potential via Gaussian summation using the electron-density map prediction process provided b PyMOL version 1.0 ("map_new" command). The process occurs in three steps: first, each grid point is mapped to a hydrogen atom with unit occupancy and a uniform isotropic temperature factor. Second, a pseudo-electron-density potential map is then computed from the hydrogen atom positions according to the atomic scattering factors formulated by Cromer and Mann) (Cromer and Mann, 1968, "X-ray scattering factors computed from numerical Hartree-Fock wave functions," Acta Cryst, A28, 321-324, which is hereby incorporated by reference herein in its entirety) and tabulated by (Wilson, 1992, International Tables for Crystallography, Volume C: Mathematical, Physical, and Chemical Tables, Kluwer Academic Publishers, London), using numerical methods adapted from X-PLOR (Brunger, 1993, X-PLOR Version 3.1: A System for X-ray Crystallography and NMR, Yale University Press, New Haven, Connecticut, U.S.A., which is hereby incorporated by reference herein). Third, the isosurface contours are drawn at constant potential levels in the computed map.

The above-identified pseudo-electron-density calculation described above is one way of recasting a set of points on a grid into a form that can be readily displayed and manipulated within a molecular modeling such as PyMOL. As such, the atomic scattering factors, the temperature factors, and the isosurface potential levels employed do not have a physical significance, but are merely parameters tuned for purposes of display. Thus, through trial-and-error, it was determined that a contour level of 1.75 potential units on a 1.0 Angstrom grid computed with a temperature factor of 30 Angstroms-squared produces a smooth volume envelope with a shape closely matching that of the input grid points. However, the user can adjust the displayed contour level as needed to expand or contract the volume within certain limits in some embodiments.

*Step 206.* In step 206, the binding volume map is further enhanced by generating an interaction map 60 for the binding site of the macromolecule. The interaction map 60 allows easy visualization of available space in a binding site and potential interactions that can be made between a ligand and the binding site. In typical embodiments, the interaction map is constructed by interaction map construction module 58. The interaction map 60 is created by characterizing regions of the binding volume map 56 by interaction types that a ligand is expected to adopt in order to achieve complementarity with the macromolecule. This can be considered a "projection" of the binding site onto the binding volume map where the properties of the binding site, in their complementary form, are projected onto the binding volume map.

The binding site volume map 56 comprises a surface that has a plurality of regions. Thus, in step 206, each respective region in the plurality of regions of the surface of the binding site volume map is classified based upon a characteristic that is complementary of a characteristic of a corresponding portion of the macromolecule, thereby creating an interaction map. In some embodiments, such assignments are performed by taking each respective grid point on the surface of the binding volume map and assigning attributes to the respective grid point based on (i) the characterization of the alpha sphere nearest the grid point (*e.g.*, where the alpha spheres have been assigned a classification of hydrophobic or hydrophilic as described above), (ii) complementarity to a property (*e.g.*, acidic, basic, hydrophobic, hydrophilic, or exposed) of a residue in the binding site that is nearest to the respective grid point, or (iii) a property of the surface (*e.g.*, electrostatic potential, hydrophobicity, acidity, *etc.*) that is closest to the respective grid point. In one example, if the corresponding portion of the macromolecule is basic, the corresponding region of the surface of the binding site volume map is classified as acidic because such a property is complementary to the basic property of the corresponding portion of the macromolecule. In another example, if the corresponding portion of the macromolecule is acidic, the corresponding region of the surface of the binding site volume map is classified as basic because such a property is complementary to the acidic property of the corresponding portion of the macromolecule. In still another example, if the corresponding portion of the macromolecule is hydrophobic, the corresponding region of the surface of the binding site volume map is classified as hydrophilic because such a property is complementary to the hydrophobic property of the corresponding portion of the macromolecule. In still another example, if the corresponding portion of the macromolecule is hydrophilic, the corresponding region of the surface of the binding site volume map is classified as hydrophobic because such a property is complementary to the hydrophilic property of the corresponding portion of the macromolecule.

In some embodiments, the amino acids histidine, lysine, and arginine are considered basic. Thus, in some embodiments, when a residue of such an amino acid is the closest residue to a grid point in an interaction map, that grid point will be assigned to be acidic in order to be complementary to the basic residue. In some embodiments, the amino acids aspartic acid and glutamic acid are considered acidic. Thus, in some embodiments, when a residue of such an amino acid is the closest residue to a grid point in an interaction map, that grid point will be assigned to be basic in order to be complementary to the acidic residue. Table 1 below lists properties of the twenty naturally occurring amino acids. In some embodiments, each grid point on the surface of a binding volume map is assigned a property that is complementary to one or more properties of the nearest residue of an amino acid in the macromolecule.

Table 1 - Properties of twenty naturally occurring amino acids

| Amino Acid | 3-letter code | 1-letter code code | Properties |
|---|---|---|---|
| Alanine | Ala | A | aliphatic hydrophobic neutral |
| Arginine | Arg | R | Polar hydrophilic charged (+) basic |
| Asparagine | Asn | N | Polar hydrophilic neutral |
| Aspartate | Asp | D | Polar hydrophilic charged (-) acidic |
| Cysteine | Cys | C | Polar hydrophilic neutral |
| Glutamine | Gln | Q | Polar hydrophilic neutral |
| Glutamate | Glu | E | Polar hydrophilic charged (-) acidic |
| Glycine | Gly | G | Aliphatic hydrophilic neutral |
| Histidine | His | H | Aromatic polar hydrophilic charged (+) basic |
| Isoleucine | Ile | I | Aliphatic hydrophobic neutral |
| Leucine | Leu | L | Aliphatic hydrophobic neutral |
| Lysine | Lys | K | Polar hydrophilic charged (+) Basic |
| Methionine | Met | M | hydrophobic neutral aromatic |
| Phenylalanine | Phe | F | hydrophobic neutral |
| Proline | Pro | P | hydrophobic neutral Polar |
| Serine | Ser | S | hydrophilic neutral Polar |
| Threonine | Thr | T | hydrophilic neutral aromatic |
| Tryptophan | Trp | W | hydrophobic neutral |
| Tyrosine | Tyr | Y | aromatic polar hydrophilic |
| Valine | Val | V | aliphatic hydrophobic neutral |

Moreover, there is no requirement in the present invention that the characterization of each respective region of the surface of the binding site volume map be classified on a strictly categorical basis (*e.g.*, hydrophilic vs. hydrophobic, basic vs. acid). In some embodiments, each respective region (*e.g.*, each grid point) of the surface of the binding site volume map is characterized by degree (*e.g.* degree of acidity, degree of hydrophobicity, *etc.*). For example, a given region on the surface may be assigned as acidic because the region of the macromolecule nearest the given region is basic. Moreover, the given region is assigned a degree of acidity (pH) based upon the degree of basicity of the region of the macromolecule nearest the given region is basic. Moreover, the such degrees (*e.g.*, degree of acidity) can be color coded and the color of each region displayed on the interaction map.

In some embodiments, the portion of the macromolecule that corresponds to a respective region of the surface of the binding site volume map (and is thus determinative of the characteristic assigned to the respective region) is one or more atoms in a residue of the macromolecule that is closest to the given region. In some embodiments, the portion of the macromolecule that corresponds to a respective region of the surface of the binding site volume map is an atom in a residue of the macromolecule that is closest to the given region.

Some examples of characteristics that can be assigned to regions of the surface of the binding site volume map to produce an interaction map have been provided. Advantageously, each respective region of the interaction map is characterized based upon a property that is complementary to the property of the portion of the macromolecule that is closest to the respective region. In this way, the interaction map provides a unique view, from an idealized ligand's perspective, of the macromolecular binding pocket.

In some embodiments, the portion of the macromolecule that corresponds to a respective region of the surface of the binding site volume map (and is thus determinative of the characteristic assigned to the respective region) is one or more atoms in a residue of the macromolecule that is closest to the given region. In some embodiments, the portion of the macromolecule that corresponds to a respective region of the surface of the binding site volume map is an atom in a residue of the macromolecule that is closest to the given region.

In some embodiments, a respective region of the binding site volume map is characterized based on any physiochemical, molecular, or structural property that is complementary to the property of the corresponding portion of the macromolecule. Examples of such physiochemical, molecular, or structural properties include, but are not limited to, electrostatic potential, hydrophilicity, acidity, and solvent accessibility, and curvature corrected solvent accessibility. Methods for computation of some of these properties are described in, for example, Honig et al., 2003, Methods Enzymol 374, 492-509, which is hereby incorporated by reference herein in its entirety.

In some embodiments, a respective region of the binding site volume map is assigned multiple characterizations based on any physiochemical, molecular, or structural properties that are complementary to corresponding properties of the corresponding portion of the macromolecule. For example, a respective region of the binding site volume map can be assigned any combination of a hydrophobicity value that represents a hydrophobicity of the respective region, an electrostatic potential value, an acidity (*e.g.*, on the pH scale), and a solvent accessibility value that represent a solvent accessibility of the respective region of the binding site volume map, thereby creating an interaction map. Then, a user can toggle between the various types of characterizations for the interaction map in order to better understand the complementarity between the interaction map (and any ligand that approximates or matches the properties of the interaction map) and the binding pocket of the macromolecule.

In some embodiments, where the grid based method described in conjunction with step 204 above is used to construct the binding site volume map, each respective portion of the binding site volume map that is characterized in order to form the interaction map is a grid point on the surface or near the surface of the binding site volume map. Further, in some embodiments, a three-dimensional surface is overlayed on the binding site volume map and each respective region of the three-dimensional surface is colored based on the underlying property of the grid point closest to the respective region. This three-dimensional surface can be, for example, the surface described in step 204 above

*Step 208.* In step 208, the interaction map is outputted, for example, to a user interface device, a monitor, a computer-readable storage medium, a computer-readable memory, or a local or remote computer system. Alternatively or additional, binding site volume map is displayed.

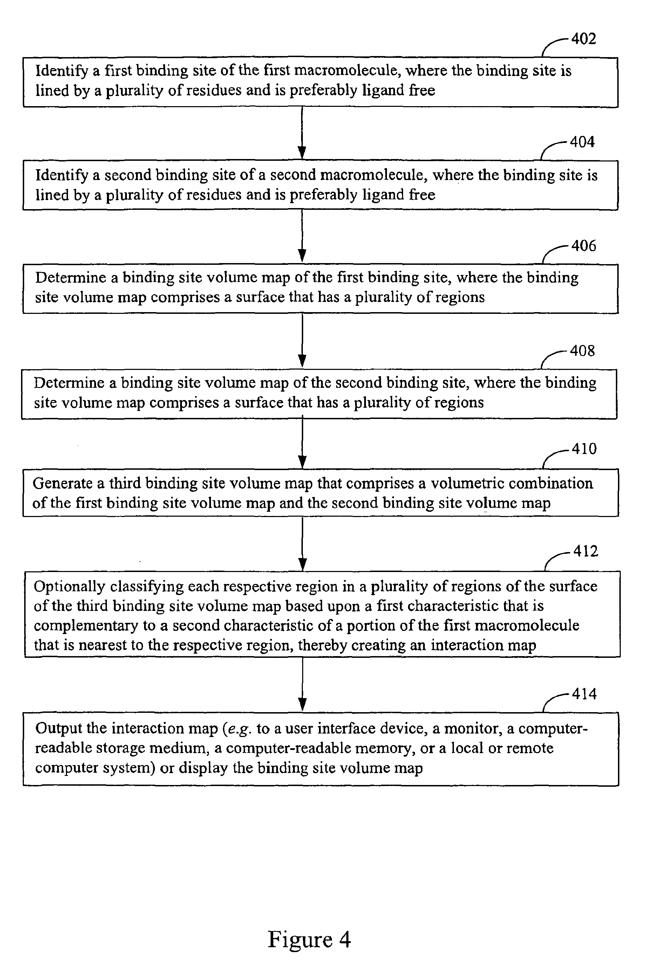

*Comparison of macromolecular binding sites.* Turning to Figure 4, an exemplary process for comparing a first binding site of a first macromolecule to a second binding site of a second macromolecule is disclosed. For ease of introducing the concepts of the present invention, the method is described for the comparison of a first macromolecule to a second macromolecule. However, in practice, there is no limit to the number of macromolecules that can be compared at the same time using the disclosed techniques.

In steps 402 and 404, the first binding site of the first macromolecule and the second binding site of the second macromolecule are identified using any of the techniques disclosed above in conjunction with step 202 of Figure 2 and/or those disclosed below in the section entitled "Exemplary Methods for Identifying a Binding Site in a Macromolecule." If additional macromolecules are to be compared, the binding sites of such macromolecules are identified as well.

In steps 404 and 406, a first binding site volume map based upon the first binding site and a second binding site volume map based upon the second binding site are determined using any of the techniques disclosed above in conjunction with step 204 of Figure 2. If additional macromolecules are to be compared, the binding site volume maps of such macromolecules are identified as well.

In step 408, a third binding site volume map is generated that comprises a volumetric combination of the first binding site volume map and the second binding site volume map. In some embodiments, such volumetric combinations are performed by composite binding site volume map 64. If additional macromolecules are to be compared, the third binding site volume map is a volumetric combination of each of the binding site volume maps of the macromolecules to be compared. In this way, binding site volume maps of different macromolecules can be compared to identify, for example, the union, intersection, difference, unique, and average regions of the binding pockets. Binding site volume maps that are formed by such operations, or combinations of such operations, are referred to herein as composite binding site maps. Such binding site volume maps are also interchangeably referred to herein as "third binding site volume maps" even if more than two binding site volume maps where used to generate such binding site volume maps.

In some embodiments, the union volume can be used to determine all grid points that fall in the volume maps of either of the macromolecules selected. In some embodiments, the union operator gives a potential map where each point contains the maximum potential value found at that same position in any of the input binding site volume maps. Thus, for any fixed contour level, this map will produce an isosurface volume consisting of the union of the volumes contoured over the input binding site volume maps. The union operator can join multiple maps together in this manner.

In some embodiments, the intersection volume is defined by grid points that are common in the volume maps of the macromolecules selected. In some embodiments, the intersection operator gives a composite binding site volume map where each point contains the minimum value found at that same position in any of the input binding site volume maps. Likewise, for any fixed contour level, this intersection map will produce an isosurface volume consisting of the intersection of the volumes contoured over the input binding site volume maps.

In some embodiments, the difference volume is a composite binding site map where the potential at each position in a binding volume site map of a first macromolecule is reduced by the potential value found at that same position in each of the binding site volume maps of other macromolecules that are being compared to the first macromolecule. When displaying such "difference" binding site volume maps, both positive and negative contours are shown. In some embodiments the "unique" operator gives the same map as the "difference" operator described above with the exception being that all negative values in the unique composite binding site volume map are truncated to zero and only the positive isosurface contour is shown. In some embodiments, an average binding site volume map gives a map where the potential value at each point represents the average value found over all of the input binding site volume maps.

Typically before the volumetric combination can be made, the binding site volume maps must be superimposed onto each other. In order to ensure proper superposition, in one embodiment, the superposition matrix that will superimpose the first and second macromolecules onto each other is typically also used to superimpose the first and second volume maps onto each other. In some embodiments, the superposition matrix that will superimpose a region around the first and second binding sites is used to superimpose the first and second binding site volume maps. In some embodiments, where the binding sites of more than two macromolecules are to be compared, the matrices that superimpose all of the macromolecules onto each other are also applied to the corresponding binding site volume maps so that they are also properly superimposed onto each other.

There are a number of ways in which the first and second macromolecule may be superimposed in order to provide an appropriate superposition matrix for the first and second binding site volume maps and all such methods are within the scope of the present invention. Such principles have likewise been extended to the alignment of more than two macromolecules but, for each of discussion, such techniques are described here for the case where just two macromolecules are to be superimposed. In some embodiments, the backbone atoms of at least the residues that line the first and second binding sites of the first and second macromolecules are superimposed onto each other and the superposition matrix derived from such a superposition is also applied to the first and second binding site volume maps so that they are also superimposed onto each other. Such a superposition typically requires an alignment of the residues in the first macromolecule with the residues in the second macromolecule. Such alignment can be done by manual inspection or by automated methods such as local (*e.g.*, Smith-Waterman algorithm, Smith and Waterman, 1981, Journal of Molecular Biology 147: 195-197, which is hereby incorporated by reference herein) or global alignment (*e.g.*, Needleman-Wunsch algorithm, Needleman and Wunsch, 1970, Journal of Molecular Biology. 48: 443-53, which is hereby incorporated by reference herein) that have been implemented in numerous publicly available software programs. Multiple sequence alignment programs are also available in the case where multiple macromolecules are to be superimposed. See, for example, Lassmann and Sonnhammer, 2005, BMC Bioinformatics 6, 298; and Higgins et al., 1994, Nucleic Acids Res. 22: 4673-4680, each of which is hereby incorporated by reference herein in its entirety.

Once each of the macromolecules has been aligned at the primary sequence level, the distance between corresponding atoms in the models of the macromolecules is minimized. One such minimization technique is the minimization of the root mean square deviation (RMSD) of corresponding atoms (*e.g.*, the corresponding atoms that form the backbone of the residues that line the first and second binding sites) using the Kabsch least-squares method (Kabsch 1978, Acta Crystallogr A 34, 827-828, which is hereby incorporated by reference herein). There is a wide range of choices for which corresponding atoms from the first and second macromolecule may be used in the alignment. For instance, all the backbone atoms of the first and second macromolecules may be used in the alignment, all atoms in first macromolecule that have a corresponding atom in the second macromolecule may be used in the alignment, and so forth. In a preferred approach a 10 Angstrom shell is drawn around each of the binding sites to be aligned and the residues (*e.g.*, backbone atoms of such residues, or all atoms in such residues that have counterparts in both molecular models) from each of the macromolecules in each of the 10 angstrom shells are used as the basis for the alignment. In other embodiments, a shell in the range between five Angstroms and 40 Angstroms is chosen based on the size of the binding pockets being analyzed and all corresponding atoms or all backbone atoms in this shell are used as the basis of the superposition.

Furthermore, there is a wide range of alignment algorithms that may be used for the superposition of the chosen set of corresponding atoms, such as a Gaussian-weighted RMSF fit using the method of Damm et al., 2006, Biophysical Journal 90, 4558-4573, which is hereby incorporated by reference herein. Software for superimposing multiple macromolecules is also available. See, for example, Petrey and Honig, 2003, Methods in Enzymology 374: 492-509, which is hereby incorporated by reference herein.

Once the binding site volume maps have been superimposed, the volumetric combination of the maps can be accomplished. In some embodiments, the volume combination is a union of the each of the binding site volume maps. In such embodiments, in the case where the third binding site volume map is a combination of a first and second binding map, the composite binding site volume map will constitute the maximal value of each spatially equivalent set of points in the first and second binding site volume maps. Thus, a surface drawn over these grid points will reflect a composite of the first and second binding site volume maps. In some embodiments, in the case where the third binding site volume map is a combination of a first, second and third, binding map and such maps comprise grid points, the composite binding site volume map will constitute the maximal value of each spatially equivalent set of points in the first, second, and third binding site volume maps. Thus, a surface drawn over these grid points will reflect a composite of the first, second, and third binding site volume maps. One of skill in the art will appreciate that such a volumetric combination map can be extended to the combination of any number of binding site volume maps (*e.g.*, three or more, four or more, ten or more, between two and one hundred, *etc.*). Such composite binding maps are useful, for example, in defining the region available in a target binding site including volume available due to flexibility of amino acid side chains.

In some embodiments, the volume combination is a difference of the first binding site volume map and the second binding site volume map. In examples of such embodiments, each position in the third binding site volume map (the composite binding site volume map) will be the value of the same position in a first binding site volume map that has been reduced by the potential value found at that same position in each of the remaining binding site volume maps. Advantageously, inspection of third binding site volume map (the composite binding site volume map) will reveal those parts of the first binding site volume map, corresponding to the first macromolecule that are not found in the binding site volume maps of the other macromolecules. Such visual inspection can be done, for example, by drawing a semi-transparent or non-transparent surface over the grid points of the third binding site volume map. First instance, such a map could be drawn using any of the techniques discussed above in conjunction with step 204. Such a map is highly advantageous for a wide range of applications such as understanding enzyme substrate selectivity and structure-based drug design. For example, the first macromolecule could be an enzyme that is to be inhibited by an inhibitor that specifically binds the first binding pocket while the second macromolecule (and subsequent macromolecules) could be an enzyme for which specific binding of the inhibitor is not desired. The third binding site volume map can be used to determine regions of the first binding pocket that are not shared by the second binding pocket (and subsequent binding pockets).

In some embodiments, the volume combination is the intersection of the first binding site volume map and the second binding site volume map. In examples of such embodiments, each position in the composite binding site volume map will be the minimum value found in the equivalent position in any of the binding site volume maps that are combined to form the composite binding site volume map. Advantageously, inspection of the grid points in the third binding site volume map will reveal those parts of the first binding site volume map that are also found in the second binding site volume map. Such visual inspection can be done, for example, by drawing a semi-transparent or non-transparent surface over the grid points of the third binding site volume map. Such a map can be drawn using the technique described above in step 204. The intersection composite binding site volume map is highly advantageous for a wide range of applications such as understanding what regions of the binding pocket do not confer selectivity. For example, the first macromolecule could be an enzyme that is to be inhibited by an inhibitor that specifically binds the first binding pocket while the second macromolecule could be an enzyme for which specific binding of the inhibitor is not desired. The composite binding site volume map can be used to determine regions of the first binding pocket that are shared by the second binding pocket and therefore do not provide potential to confer enzyme selectivity.

Other volumetric combinations of the first binding site volume maps are possible. For instance, the volumetric combination can be an average of two or more binding site volume maps. In another example, the volumetric combination is a uniqueness function in which the third binding site volume map consists of (i) those portions of the first binding site volume map that are not shared by the second binding site volume map; and (ii) those portions of the second binding site volume map that are not shared by the first binding site volume map. In some embodiments, the first and second binding site volume maps constitute grid points and the aforementioned portions are identified by determining, for each grid point in each respective binding site volume map, whether there is an equivalent grid point within the grid spacing in the other binding site volume map. In such an instance the composite binding volume map highlights the region unique to the first binding site. Ligands occupying part or whole of this region are expected to be selective for the first binding site.

Still other volumetric combinations of maps are possible. For instance, in some embodiments, the volumetric combination can be the difference between (i) the union of a first and second binding site volume map and (ii) a third second binding site volume map. In another instance, in some embodiments, the volumetric combination can be the difference between (i) the intersection of a first and second binding site volume map and (ii) a third second binding site volume map. Given the disclosure herein, one of skill in the art will appreciate that the composite binding site volume maps generated by volumetric combination module 62 can be any logical combination of two or more binding site volume maps. Furthermore, such composite binding site volume maps can themselves be logically combined using, for example, the union operator, the intersection operator, the difference operator, and so forth. Furthermore, the binding site volume maps can be stored in computer 10 or a computer addressable by computer 10 for later use and analysis.

In step 412, the each respective region (*e.g.*, respective grid point) in a plurality of regions of the surface of the third binding site volume map (the composite binding site volume map) is optionally classified based upon a first characteristic that is complementary to a second characteristic of a portion of the first macromolecule that is nearest to the respective region (*e.g.*, the residue in the first macromolecule that is closest to the respective grid point) thereby creating an interaction map for the third binding site volume map. Any of the classification techniques described above in conjunction with step 206 of Figure 2 can be used in such a classification. In some embodiments, advantageously, such an interaction map not only highlights portions of the binding pockets that are shared or unique to two macromolecules, it also provides a convenient means for visualizing the molecular properties that a ligand should have in order to exploit this uniqueness. For instance, in the case where the interaction map highlights the difference between the first and second macromolecule, the interaction map not only graphically highlights regions that can be exploited by a ligand for selectivity but also illustrates what molecular properties such a ligand should have.

In step 414, the third binding site volume map (the composite binding site volume map) and/or the interaction map are outputted to a user interface device, a monitor, a computer-readable storage medium, a computer-readable memory, or a local or remote computer system. Alternatively, the third binding site volume map and/or the interaction map are displayed.

The binding site volume maps and interaction maps of the present invention can be visualized by any number of commercially available and publicaly available software programs including, but not limited to, Gaussian 92 (Frisch, Gaussian, Inc., Pittsburgh, Pennsylvania) AMBER (Kollman, University of California at San Francisco); QUANTA/ CHARMM (Molecular Simulations, Inc., Burlington, Massachusetts), Insight II/Discover (Biosym Technologies Inc., San Diego, California) a MOE Isosurface, or a Pymol (DeLano, "The PyMOL Molecular Graphics System," DeLano Scientific LLC, Palo Alto, California) Gaussian surface. Both the grid points that fill the binding pocket and any three-dimensional surface drawn over such grid points may be referred to herein, interchangeably, as the binding volume map 56. Furthermore, any other three-dimensional representation of the binding site 50 is deemed to be a binding site volume map 56. Both the characterized grid points that fill the binding pocket and any three-dimensional surface drawn over such grid points may be referred to herein, interchangeably, as the interaction map 60.

*Exemplary Methods for Identifying a Binding Site in a Macromolecule*

Methods for identifying a binding site of macromolecule include, but are not limited to, the following approaches: energetic based methods, grid based methods, geometric methods, classification methods, and direct methods, as well as any combination thereof. Nonlimiting examples of such methods are disclosed below.

*Energetic methods.* In methods such as Goodford, 1985, J. Med. Chem. 28, 849-857, and Miranker, 1991, Proteins: Structure, Function, and Genetics 11, 29-34, each of which is hereby incorporated by reference herein, interaction energies between the target protein and different probes are computed in an attempt to locate energetically favorable sites. Such energetic procedures typically require the assignment of proton locations and partial charges to the receptor atoms.

*Grid based methods.* One example of a grid base method is Ligsite (Hendlich et al., 1997, Journal of Molecular Graphics and Modeling 15, 359-363, hereby incorporated by reference herein). Pockets are identified with a series of operations on a cubic grid.

*Geometric methods.* Yet another approach to identifying binding sites of macromolecules is the use of geometric methods. Geometric methods require the three-dimensional coordinates of the target macromolecule. Such methods explore the surface of the target macromolecule without the use of energy models. There are a number of different types of geometric methods. Geometric methods include, but are not limited to, the implementation found in the Molecular Operating Environment (MOE) (*e.g.*, Version 2005.06 and later), which is distributed by the Chemical Computing Group (Montreal, Quebec Canada H3A 2R7), ProShape (Edelsbrunner and Koehl, 2003, Proc. Natl. Acad. Sci. USA 100: 2203-2208, which is hereby incorporated by reference herein), CAST (Prompanas et al., 2000, Bioinformatics 16: 915-922, which is hereby incorporated by reference herein) and the analytic geometric algorithms of Del Carpio et al., 1992, J. Mol. Graphics 11, 23-29, which is hereby incorporated by reference herein. Additional geometric methods include those disclosed by Kuntz et al., 1982, J. Mol. Biol. 161, 269-288, Ho and Marshall, 1990, J. Comput.-Aided Mol. Design 4, 337-354, and Bardford et al., 1988, Biochemistry 27, 6733-6741, each of which is hereby incorporated by reference herein. In yet another approach to determining binding site of a macromolecule, a neural network based protocol is used to identify cavities on the surface of the target sequence. These cavities are considered potential binding sites (Stahl & Schneider, 2000, Protein Engineering, 13, 83-99, which is hereby incorporated by reference herein). The neural network approach has been applied to a set of 176 zinc metalloproteinases. In most, but not all cases, the actual binding site of the target macromolecule was represented by one of the five largest cavities on the surface of the molecule. In a similar method used to predict the active site residues from the three-dimensional representation corresponding to a target sequence, descriptors of protein active sites, termed "fuzzy functional forms" (FFFs), have been defined (Fetrow & Skolnick, 1998, Journal of Molecular Biology 281, 949-968, which is hereby incorporated by reference herein). FFFs are derived from the geometry, residue identity, and conformation of protein active sites in known x-ray crystal structures of proteins. FFFs are used to identify a macromolecular binding site in the ab initio or the threading models of various macromolecules. In the case of proteins, in one approach, algorithms have been developed to use the structure of a target sequence in order to predict the enzyme class of the target sequence. For example, an algorithm called TESS has been developed to search for user-defined spatial combinations of atoms in the Protein Data Bank (PDB) (Wallace et al., 1997, Protein Science 6, 2308-2323, which is hereby incorporated by reference herein). The PDB is a publicly available database of three-dimensional representations of proteins that have been derived by techniques such as two- and three-dimensional nuclear magnetic resonance as well as x-ray crystallography. TESS derives three-dimensional templates from three-dimensional representations deposited in the PDB. Using TESS, a new structure that corresponds to the target sequence is scanned against these three-dimensional templates in order to determine the binding site of the new structure

*Classification methods.* Example of classification methods include, but are not limited to, SURFNET (Laskowski, 1995, Journal of Molecular Graphics 13, 323-330), PocketPicker (Weisel et al., 2007, Chemistry Central Journal 1:7), POCKET (Levitt, 1992, J. Mol. Graphics 10, 229-234), and the Cellular Logic Operations of Delaney (Delaney, 1992, J. Mol. Graphics 10, 174-177). Still another classification method is the computational tool called PASS (Lagunin et al., 1999, Bioinformatics 16: 747-748). PASS characterizes regions of buried volume in target sequences following approaches similar to the neural network approach of Stahl & Schneider (Brady & Stouten, 2000, Journal of Computer-Aided Molecular Design 14, 383). Still another approach to determining the binding site of a target macromolecule is three-dimensional cluster analysis (Landgraf et al., 2001, Journal of Molecular Biology 307, 1487). Three-dimensional cluster analysis identifies active site residues by taking into account the conservation of spatially defined residue clusters within the target sequence relative to the target sequence as a whole.

*Direct methods.* A direct method for determining the binding site of a macromolecule is to solve the three-dimensional structure of the protein corresponding to the sequence complexed with a ligand that binds to the binding site of the protein. A number of proteins have been determined in such complexes using x-ray crystallographic or nuclear magnetic resonance techniques. The identity of the residues that form the binding site in a particular protein that has been solved by such techniques provides a source of information that can be used to predict which residues will form the binding site of another protein. To exploit this form of information, Stuart et al. (Stuart, 2001, LigBase: a database of families of aligned ligand binding sites in known protein sequences and structures, Bioinformatics 17, 1-2) developed a database that contains all binding sites of known structures. Furthermore, for each protein of known structure, the database provides an alignment with all related protein sequences and structures. The LigBase sequence alignments can be used to predict the binding site residues of proteins that are similar to proteins that have known complexed structures.

*Exemplary sequence alignment programs*

Existing sequence comparison processes used in some embodiments of processing step 410 (Figure 4) may be divided into two main classes: global comparison methods and local comparison methods. In global comparison methods, sequences are aligned in their entirety and scored in a single operation (Needleman and Wunsch, 1970, Molecular Biology 48, 443). In local comparison methods, only highly similar segments of two sequences are aligned and scored, and a composite score is computed by combining the individual segment scores, *e.g.*, the FASTA method (Pearson and Lipman, 1988, Proc. Natl. Acad. Sci. USA 85: 2444-2448), the BLAST method (Altschul, et al., 1990, Journal of Molecular Biology 215, 403-410; Altschul et al., 1997, Nucleic Acids Research 25, 3389-3402) and the BLAZE method (Brutlag, et al., 1993, Computational Chemistry 17, 203-207).

In some embodiments, sequence alignment of macromolecules is performed using an algorithm such as Basic Local Alignment Search Tool (BLAST), PSI-BLAST, PHI-BLAST, WU-BLAST-2, and/or MEGABLAST. See Altschul et al., 1990, J. Mol. Biol. 215, 403-410; Altschul et al., 1996, Methods in Enzymology 266, 460-480; and Karlin et al., 1993, PNAS USA 90, 5873-5787. Additional algorithms that may be used to align macromolecules include FASTA (Pearson, 1995, Protein Science 4, 1145-1160), ClustalW (Higgin et al., 1996, Methods Enzymol. 266, 383-402), DbClustal (Thompson et al., 2000, Nucl. Acids Res. 28, 2910-2926), and the Molecular Operating Environment (Chemical Computing Group, Montreal, Quebec Canada H3A 2R7).

Various multiple sequence alignment programs include, but are not limited to, FASTA (Pearson, 1995, Protein Science 4, 1145-1160), ClustalW (Higgin et al., 1996, Methods Enzymol. 266, 383-402), MSF (European Molecular Biology Laboratory, Meyerhofstr. 1, 69117 Heidelberg, Germany), as well as Modeler's PIR format (Sali and Sanchez, 2000, Methods Mol. Biol. 143, 97-129).

In some embodiments, where the sequence to be aligned are proteins, such sequence alignments include a pairwise alignment using an amino acid substitution matrix (*e.g.*, an alignment scoring table). An amino acid substitution matrix provides a numerical score for each of the possible pairings or substitutions that can be found at individual residue positions in an alignment. It will be appreciated that, in one embodiment, the amino acid substitution matrix is a 20 x 20 matrix, where elements of the matrix represent the score for substituting one of the naturally occurring amino acids with another of the naturally occurring amino acids. In some embodiments, a BLOSUM62 matrix is the amino acid substitution matrix used by the alignment module algorithm. The BLOSUM62 matrix is a derivative of the Dayhoff scoring matrix. The Dayhoff matrix provides a numerical value for substitution from any one of the twenty naturally occurring amino acids to another amino acid. (See Henikoff & Henikoff, 1993, Proteins 17, 49-61, 1993). While any amino acid substitution matrix can be used, additional illustrative matrices are presented herein. For example, the WAC matrix (Pac. Symp. Biocomput., 465-76, 1997) can be used. The WAC matrix is the result of a comprehensive analysis of the microenvironments surrounding the twenty naturally occurring amino acids. This analysis includes a comparison of amino acid environments with random control environments as well as with each of the other amino acid environments. These environments are described with a set of 21 features summarizing atomic, chemical group, residue, and secondary structural features. The environments are divided into radial shells of one Angstrom thickness to represent the distance of the features from the amino acid $C_\alpha$ atoms. Still another amino acid substitution matrix is a Risler matrix (Risler et al., 1988, J. Mol. Biol. 204, 1019-29). In developing the Risler matrix, an amino acid a, in a protein PI is considered replaced by the amino acid $a_2$ in the structurally similar protein P2 when, after superposition of the two structures, the $a_1$ and $a_2$ $C_\alpha$. atoms are no more than 1.2 Angstroms apart. Using this criterion, amino acid pairs (substitutions) from various structures were analyzed by statistical methods to produce the Risler matrix.

Another non-limiting example of an alignment program that can be used to compare the sequences of macromolecules is the algorithm of Myers and Miller (Myers & Miller, CABIOS 4, 11-17, 1988). Such an algorithm is incorporated into the ALIGN program (version 2.0) which is part of the GCG sequence alignment software package. In some instances, when utilizing the ALIGN program for comparing amino acid sequences, a PAM120 alignment scoring table (Henikoff & Henikoff, 1992, Proc. Natl. Acad. Sci. USA, 89, 10915), a gap length penalty of 12, and a gap penalty of 4 can be used. Additional algorithms for sequence analysis are known in the art and include, but are not limited to, ADVANCE and ADAM (Torellis & Robotti, 1994, Comput. Appl. Biosci., 10:3-5). Many other amino acid substitution matrices can be used. Such tables include, but are not limited to the PAM250 matrix (Henikoff & Henikoff, 1992, Proc. Natl. Acad. Sci. USA 89, p. 10915).

*Computer and Computer Program Product Implementations.*

The present invention can be implemented as a computer program product that comprises a computer program mechanism embedded in a computer-readable storage medium. Further, any of the methods of the present invention can be implemented in one or more computers. Further still, any of the methods of the present invention can be implemented in one or more computer program products. Some embodiments of the present invention provide a computer program product that encodes any or all of the methods disclosed herein. Such methods can be stored on a CD-ROM, DVD, magnetic disk storage product, or any other computer-readable data or program storage product. Such methods can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. Such methods encoded in the computer program product can also be distributed electronically, via the Internet or otherwise, by transmission of a computer data signal (in which the software modules are embedded) either digitally or on a carrier wave.

Some embodiments of the present invention provide a computer program product that contains any or all of the program modules shown in Figure 1. These program modules can be stored on a CD-ROM, DVD, magnetic disk storage product, or any other computer-readable data or program storage product. The program modules can also be embedded in permanent storage, such as ROM, one or more programmable chips, or one or more application specific integrated circuits (ASICs). Such permanent storage can be localized in a server, 802.11 access point, 802.11 wireless bridge/station, repeater, router, mobile phone, or other electronic devices. The software modules in the computer program product can also be distributed electronically, via the Internet or otherwise, by transmission of a computer data signal (in which the software modules are embedded) either digitally or on a carrier wave.

## EXAMPLES

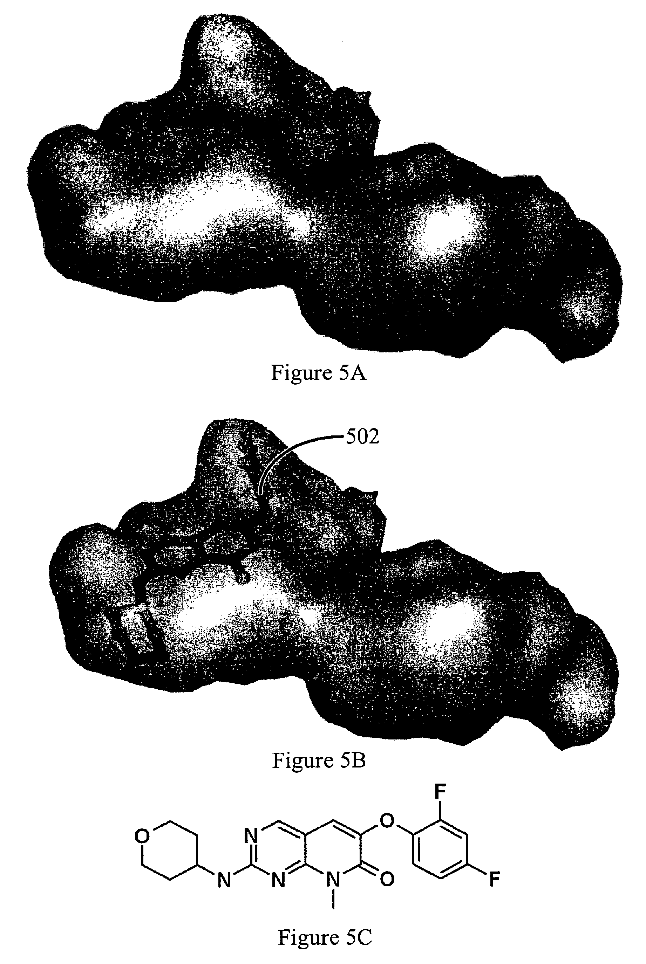

Referring to Figure 5A, the binding site of the P38A kinase (*see* Trejo et al., 2003, J. Med. Chem 46, 4702-4713, which is hereby incorporated by reference herein) was compared to the binding site of the JNK3 kinase (Weston and Davis, 2002, Curr. Opin. Genet. Dev. 12, 14-21, which is hereby incorporated by reference herein). The active site (adenosine triphosphate binding site) of P38A and JNK3 was identified using a modified MOE Alpha Site Finder. Then a binding site volume map was constructed for both P38A and JNK3 using the systems and methods described above. Next, a volumetric combination of the binding site volume map of P38A and the binding site volume map of JNK3 was generated. Specifically, the intersection of the binding site volume map of P38A and the binding site volume map of JNK3 was generated and is illustrated in Figure 5A. The intersection volume illustrated in Figure 5A defines the common allowed volume for targeting both the P38A and JNK3 binding sites. Figure 5B illustrates the inhibitor R1487 (element 502 of Figure 5B) in this intersection volume. Figure 5C provides the molecular structure of R1487. As illustrated in Figure 5B, the inhibitor is fully contained within the intersection volume. Thus, it is expected that this inhibitor will specifically bind to both P38A and JNK3 and will not exhibit selectivity between the two kinases. In fact, this is the case. The $IC_{50}$ of R1487 for P38A and JNK2 is 0.010 $\mu$M and 0.200 $\mu$M, respectively.

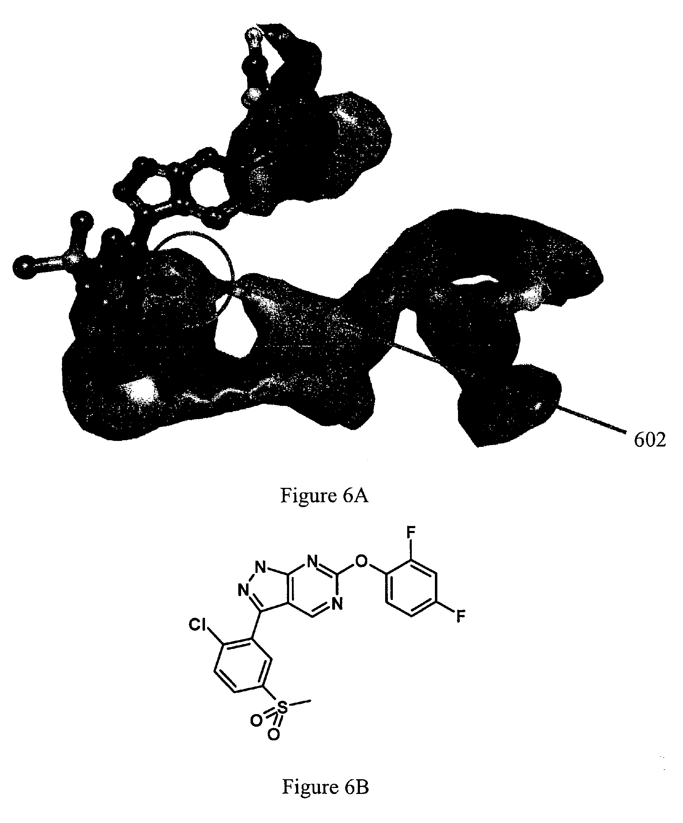

Referring to Figure 6A, the binding site of the P38A kinase was compared to the binding site of the JNK kinase. Here, however, the volumetric combination of the binding site volume maps of P38A and JNK3 were generated as a difference map. The difference map illustrated in Figure 6 defines the regions that are present in P38A binding site volume map but are not present in the JNK binding site volume map. As illustrated in Figure 6, the inhibitor RO7125 places a chlorophenyl group into region 602 that is unique to the P38A binding pocket. Thus, it is expected that RO7125 will specifically bind to P38A but not JNK and thus will exhibit selectivity to P38A over JNK. In fact, this is the case. The $IC_{50}$ of RO7125 for P38A and JNK2 is 0.006 $\mu$M and > 20 $\mu$M, respectively. The molecular structure of RO7125 is given in Figure 6B.

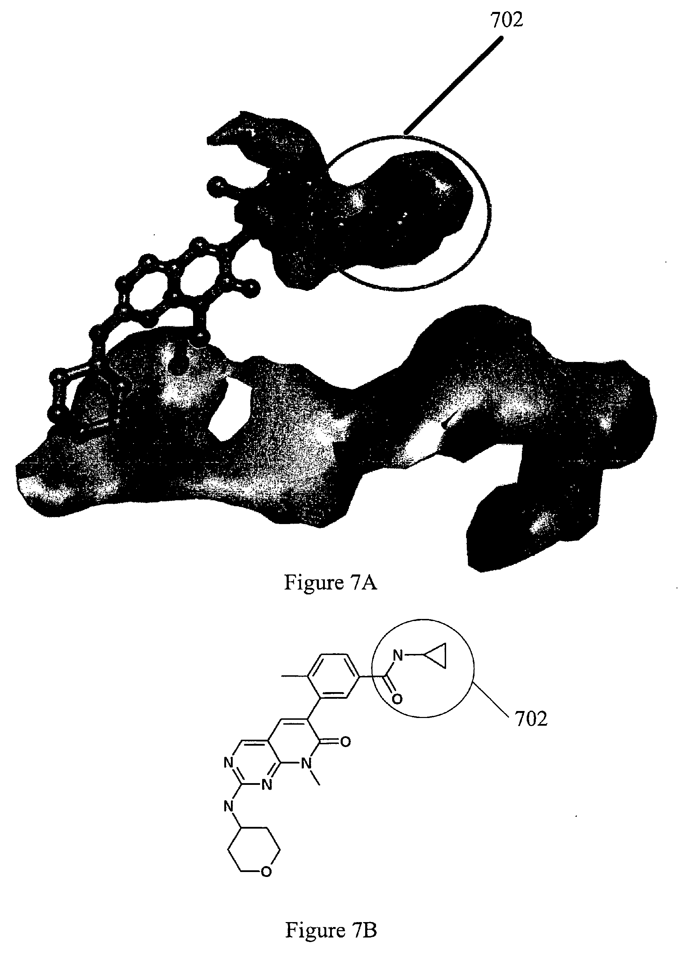

Referring to Figure 7A, the binding site of the P38A kinase was compared to the binding site of the JNK kinase. Here, the volumetric combination of the binding site volume maps of P38A and JNK were generated as a difference map. The difference map illustrated in Figure 7A defines the regions that are present in the P38A binding site volume map but are not present in the JNK binding site volume map. As illustrated in Figure 7A, the inhibitor RO9552 places a cyclopropyl group into region 702 that is unique to the P38A binding pocket. Thus, it is expected that RO9552 will specifically bind to P38A but not JNK and thus will exhibit selectivity to P38A over JNK. In fact, this is the case. The $IC_{50}$ of RO9552 for P38A and JNK2 is 0.0009 $\mu$M and 0.05 $\mu$M, respectively. Figure 7B illustrates the molecular structure of RO9552. In Figure 7B, the portion of the molecular structure that occupies region 702 of the P38A binding pocket is similarly identified as element 702.

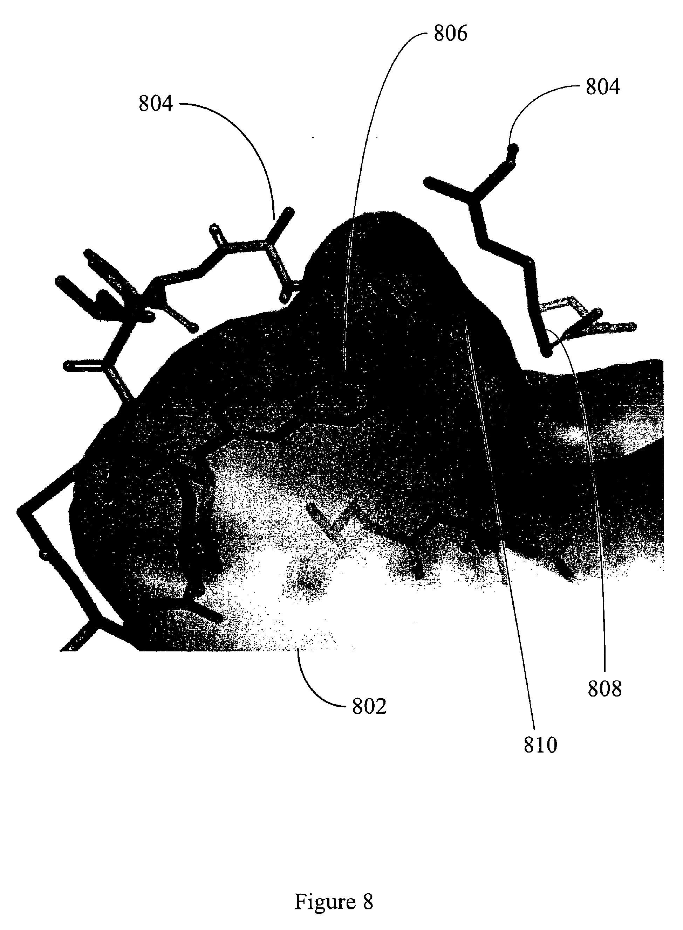

Referring to Figure 8, the binding site of the P38A kinase is evaluated by identifying the binding site of the kinase and then determining a binding site volume map of the binding site using the systems and methods disclosed above. In Figure 8, elements 804 are portions of the P38A residues that line the P38A binding pocket. Then, using the systems and methods disclosed above, each respective region in the plurality of regions of the surface of the binding site volume map is classified based upon a first characteristic that is complementary to a second characteristic of a portion of the macromolecule that is nearest to the respective region, thereby creating an interaction map 802. In the P38A binding site, a lysine 808 is near a region of the interaction map and thus the region 810 of the interaction map 802 nearest this lysine 808 is characterized as acidic. The inhibitor R06257 (element 806 in Figure 8) is superimposed into the interaction map 802. The inhibitor R06257 does not have a suitable polar atom to interact with lysine 808. Thus, RO6257 exhibits an $IC_{50}$ of only 0.25 $\mu$M against P38A. From interaction map 802, it is expected that the specificity of the inhibitor could be improved by positioning one or more polar atoms that would interaction with basic lysine 808.

## REFERENCES CITED

All references cited herein are incorporated herein by reference in their entirety and for all purposes to the same extent as if each individual publication or patent or patent application was specifically and individually indicated to be incorporated by reference in its entirety herein for all purposes.

## MODIFICATIONS

Many modifications and variations of this invention can be made without departing from its spirit and scope, as will be apparent to those skilled in the art. The specific embodiments described herein are offered by way of example only, and the invention is to be limited only by the terms of the appended claims, along with the full scope of equivalents to which such claims are entitled.

## Claims

1. A method of evaluating a binding site of a macromolecule, the method comprising:

    (A) identifying the binding site of the macromolecule, wherein the binding site is lined by a plurality of residues and wherein the binding site is ligand free;
    (B) determining a binding site volume map of the binding site, wherein the binding site volume map comprises a surface that has a plurality of regions; and
    (C) classifying each respective region in the plurality of regions of the surface of the binding site volume map based upon a first characteristic that is complementary to a second characteristic of a portion of the macromolecule that is nearest to the respective region, thereby creating an interaction map.

2. The method of claim 1, wherein the macromolecule is a protein; or wherein the macromolecule is a nucleic acid, a peptide, a protein-protein complex, or a protein-nucleic acid complex.

3. The method of claim 1 or 2, wherein the identifying (A) comprises searching a model of the macromolecule for the binding site using an energetic function, a grid based algorithm, a geometric algorithm, a classification method, or a direct method.

4. The method of any of claims 1 to 3, wherein the second characteristic of a respective portion of the macromolecule that is nearest to a respective region in the plurality of regions of the surface of the binding site volume map is:

   a hydrophobicity of one or more atoms in the residue in the plurality of residues that is nearest the respective portion of the macromolecule;
   an amount of solvent exposure of one or more atoms in the residue in the plurality of residues that is nearest the respective portion of the macromolecule; or
   a classification of each of one or more atoms in the residue in the plurality of residues that is nearest the respective portion of the macromolecule as acidic, basic, hydrophobic, or solvent exposed.

5. The method of any of claims 1 to 3, wherein the first characteristic is basic and the second characteristic is acidic; or wherein the first characteristic is acidic and the second characteristic is basic; or wherein the first characteristic is hydrophilic and the second characteristic is hydrophobic; or wherein the first characteristic is hydrophobic and the second characteristic is hydrophilic.

6. The method of any of claims 1 to 5, wherein the classifying of a respective region comprises coloring the respective region based upon the first characteristic assigned to the respective region.

7. The method of claim 1, wherein the identifying (A) comprises:

   (i) determining the Voronoi region of each atom of the macromolecule, thereby determining a plurality of Voronoi regions;
   (ii) determining each Voronoi vertex in the plurality of Voronoi regions, thereby determining a plurality of Voronoi vertices;
   (iii) assigning an alpha sphere to each Voronoi vertex in the plurality of Voronoi vertices, thereby determining a plurality of alpha spheres;
   (iv) classifying each respective sphere in the plurality of alpha spheres as a hydrophilic sphere or a hydrophobic sphere based on an ability of the respective sphere to form a hydrogen bond with an atom of the macromolecule;
   (v) clustering the plurality of alpha spheres thereby forming a plurality of candidate binding sites, wherein each respective candidate binding site in the plurality of candidate binding sites comprises three or more alpha spheres in the plurality of alpha spheres, wherein at least one alpha sphere in the three or more alpha spheres is hydrophobic;
   (vi) ranking each respective candidate binding site in the plurality of candidate binding sites by a number of hydrophobic contacts made by the respective candidate binding site to the macromolecule, thereby forming a ranked set of candidate binding sites;
   (vii) determining the binding site from the ranked set of candidate binding sites; and the method optionally further comprising eliminating near duplicate alpha spheres in the plurality of alpha spheres prior to said clustering (v).

8. The method of claim 7, wherein the determining (vii) comprises
   receiving a selection of a candidate binding site in the plurality of candidate binding sites from a user, wherein the candidate binding site selected by the user is deemed to be the binding site, or
   deeming the candidate binding site in the ranked set of candidate binding sites that has a maximum number of hydrophobic contacts with the macromolecule to be the binding site.

9. The method of claim 7, wherein the clustering (v) comprises single-linkage clustering of the plurality of alpha spheres.

10. The method of claim 1, wherein the determining (B) comprises:

   (i) placing a grid on the alpha spheres in the binding site, wherein the grid comprises a plurality of grid points; and
   (ii) identifying the binding site volume map as all grid points in the grid that both (a) fall inside the alpha spheres of the binding site map and (b) are within a predetermined distance of at least one atom of the macromolecule; optionally wherein the predetermined distance is a distance between 1.2 Angstroms and 2.0 Angstroms, or wherein the predetermined distance is between 1.35 Angstroms and 1.85 Angstroms.

**11.** A method of comparing a first binding site of a first macromolecule to a second binding site of a second macromolecule, the method comprising:

(A) identifying the first binding site of the first macromolecule, wherein the first binding site is ligand free;
(B) identifying the second binding site of the second macromolecule, wherein the second binding site is ligand free;
(C) determining a first binding site volume map based upon the first binding site;
(D) determining a second binding site volume map based upon the second binding site; and
(E) generating a third binding site volume map that comprises a volumetric combination of the first binding site volume map and the second binding site volume map.

**12.** The method of claim 11, wherein the first macromolecule is a first protein and the second macromolecule is a second protein; or wherein the first macromolecule is a nucleic acid, a peptide, a protein-protein complex, or a protein-nucleic acid complex and the second macromolecule is a nucleic acid, a peptide, a protein-protein complex, or a protein-nucleic acid complex.

**13.** The method of claim 11, wherein the identifying (A) comprises searching a first model of the first macromolecule for said first binding site using an energetic function, a grid based algorithm, a geometric algorithm, a classification method, or a direct method.

**14.** The method of claim 11, wherein the identifying (B) comprises searching a second model of the second macromolecule for said second binding site using an energetic function, a grid based algorithm, a geometric algorithm, a classification method, or a direct method.

**15.** The method of claim 11, wherein the volumetric combination is:

(a) a union of the first binding site volume map and the second binding site volume map,
(b) a difference of the first binding site volume map and the second binding site volume map,
(c) an intersection of the first binding site volume map and the second binding site volume map, or
(d) an average of the first binding site volume map and the second binding site volume map.

**16.** The method of claim 11, wherein the volumetric combination is a uniqueness function and wherein the third binding site volume map consists of (i) those portions of the first binding site volume map that are not shared by the second binding site volume map; and (ii) those portions of the second binding site volume map that are not shared by the first binding site volume map.

**17.** The method of claim 11, wherein the identifying (A) comprises:

(i) determining the Voronoi region of each atom of the first macromolecule, thereby determining a plurality of Voronoi regions;
(ii) determining each Voronoi vertice in the plurality of Voronoi regions, thereby determining a plurality of Voronoi vertices;
(iii) assigning an alpha sphere to each Voronoi vertice in the plurality of Voronoi vertices, thereby determining a plurality of alpha spheres;
(iv) classifying each respective sphere in the plurality of alpha spheres as a hydrophilic sphere or a hydrophobic sphere based on an ability of the respective sphere to form a hydrogen bond with an atom of the macromolecule;
(v) clustering the plurality of alpha spheres thereby forming a plurality of candidate binding sites, wherein each respective candidate binding site in the plurality of candidate binding sites comprises three or more alpha spheres in the plurality of alpha spheres, wherein at least one alpha sphere in the three or more alpha spheres is hydrophobic;
(vi) ranking each respective candidate binding site in the plurality of candidate binding sites by a number of hydrophobic contacts made by the respective candidate binding site to the macromolecule, thereby forming a ranked set of candidate binding sites;
(vii) determining the first binding site from the ranked set of candidate binding sites; and the method optionally further comprising eliminating near duplicate alpha spheres in the plurality of alpha spheres prior to said clustering (v).

**18.** The method of claim 17, wherein the determining (vii) comprises:

(a) receiving a selection of a candidate binding site in the plurality of candidate binding sites from a user, wherein the candidate binding site selected by the user is deemed to be the first binding site, or

(b) deeming the candidate binding site in the ranked set of candidate binding sites that has a maximum number of hydrophobic contacts with the macromolecule to be the first binding site.

19. The method of claim 17, wherein the clustering(v) comprises single-linkage clustering of the plurality of alpha spheres.

20. The method of claim 11, the determining (C) comprises:

(i) placing a grid on the alpha spheres in the first binding site, wherein the grid comprises a plurality of grid points; and

(ii) identifying the first binding site volume map as all grid points in the grid that both (a) fall inside the alpha spheres of the binding site map and (b) are within a predetermined distance of at least one atom of the first macromolecule; optionally wherein the predetermined distance is a distance between 1.2 Angstroms and 2.0 Angstroms, or wherein the predetermined distance is between 1.35 Angstroms and 1.85 Angstroms.

21. The method of claim 11, wherein the method further comprises classifying each respective region in a plurality of regions of the surface of the third binding site volume map based upon a first characteristic that is complementary to a second characteristic of a portion of the first macromolecule that is nearest to the respective region, thereby creating an interaction map.

22. The method of claim 11, wherein the third binding site volume map comprises a volumetric combination of the binding site volume map of more than two macromolecules; or wherein the third binding site volume map comprises a volumetric combination of the binding site volume map of more than five macromolecules; or wherein the third binding site volume map comprises a volumetric combination of the binding site volume map of more than fifteen macromolecules.

23. The method of claim 1, the method further comprising:

(D) outputting the interaction map to a user interface device, a monitor, a computer-readable storage medium, a computer-readable memory, or a local or remote computer system; or displaying the binding site volume map.

24. The method of claim 11, the method further comprising:

(F) outputting the third binding site volume map to a user interface device, a monitor, a computer-readable storage medium, a computer-readable memory, or a local or remote computer system; or displaying the third binding site volume map.

25. A computer-readable medium storing a computer program product, executable by a computer, to evaluate a binding site of a macromolecule, the computer program product comprising instructions for carrying out the method of any one of claims 1-24.

26. An apparatus for evaluating a binding site of a macromolecule, the apparatus comprising:

a processor; and

a memory, coupled to the processor, the memory storing a module comprising instructions for carrying out the method of any one of claims 1-24.

Figure 1

202

Identify the binding site of the macromolecule, where the binding site is lined by a plurality of residues and is preferably ligand free

204

Determine a binding site volume map of the first binding site, where the binding site volume map comprises a surface that has a plurality of regions (e.g. grid points)

206

Classify each respective region (*e.g.* respective grid point) in the plurality of regions of the surface of the binding site volume map based upon a characteristic that is complementary to a characteristic of a corresponding portion of the macromolecule (e.g. the residue in the macromolecule that is closest to the respective region), thereby creating an interaction map

208

Output the interaction map (*e.g.* to a user interface device, a monitor, a computer-readable storage medium, a computer-readable memory, or a local or remote computer system) or display the binding site volume map

# Figure 2

Figure 3

/‑402

Identify a first binding site of the first macromolecule, where the binding site is lined by a plurality of residues and is preferably ligand free

/‑404

Identify a second binding site of a second macromolecule, where the binding site is lined by a plurality of residues and is preferably ligand free

/‑406

Determine a binding site volume map of the first binding site, where the binding site volume map comprises a surface that has a plurality of regions

/‑408

Determine a binding site volume map of the second binding site, where the binding site volume map comprises a surface that has a plurality of regions

/‑410

Generate a third binding site volume map that comprises a volumetric combination of the first binding site volume map and the second binding site volume map

/‑412

Optionally classifying each respective region in a plurality of regions of the surface of the third binding site volume map based upon a first characteristic that is complementary to a second characteristic of a portion of the first macromolecule that is nearest to the respective region, thereby creating an interaction map

/‑414

Output the interaction map (*e.g.* to a user interface device, a monitor, a computer-readable storage medium, a computer-readable memory, or a local or remote computer system) or display the binding site volume map

# Figure 4

Figure 5A

Figure 5B

Figure 5C

Figure 6A

Figure 6B

702

Figure 7A

702

Figure 7B

Figure 8

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 82369707 A
- RO 7125

**Non-patent literature cited in the description**

- **RUTENBER et al.** *J. Biol. Chem.,* 1993, vol. 268, 15343-15346
- **GHOSH et al.** *J. Med. Chem.,* 1994, vol. 37, 2506-2508
- **LAM et al.** *Science,* 1994, vol. 263, 380-384
- **LEVITT et al.** *J. Mol. Graphics,* 1992, vol. 10, 229-234
- **VOORINTHOLD et al.** *J. Mol. Graphics,* 1989, vol. 7, 243 245
- **DELANEY et al.** *J. Mol. Graphics,* 1992, vol. 10, 174 177
- **DEL CARPIO et al.** *J. Mol. Graphics,* 1993, vol. 11, 23 29
- **KISLJUK et al.** *J. Mol. Graphics,* 1994, vol. 12, 305 307
- **MASUYA et al.** *J. Mol. Graphics,* 1995, vol. 13, 331 336
- **LASKOWSKI et al.** *J. Mol. Graphics,* 1995, vol. 13, 323 330
- **HENDLICH et al.** *J. Mol. Graphics,* 1998, vol. 15, 359-363
- **IRVING et al.** *Proteins,* 2001, vol. 42, 378-382
- **EDELSBRUNNER et al.** *Proc. of the 28th Annual Hawaii Intl. Conf. on Systems Science,* 1995, 256-264
- **CONNOLLY.** *J. Appl. Cryst.,* 1983, vol. 16, 548-558
- **DELANO.** The PyMOL Molecular Graphics System. DeLano Scientific LLC
- **CROMER ; MANN.** X-ray scattering factors computed from numerical Hartree-Fock wave functions. *Acta Cryst,* 1968, vol. A28, 321-324
- **WILSON.** International Tables for Crystallography, Volume C: Mathematical, Physical, and Chemical Tables. Kluwer Academic Publishers, 1992, vol. C
- **BRUNGER.** X-PLOR Version 3.1: A System for X-ray Crystallography and NMR. Yale University Press, 1993
- **HONIG et al.** *Methods Enzymol,* 2003, vol. 374, 492-509
- **SMITH ; WATERMAN.** *Journal of Molecular Biology,* 1981, vol. 147, 195-197

- **NEEDLEMAN ; WUNSCH.** *Journal of Molecular Biology,* 1970, vol. 48, 443-53
- **LASSMANN ; SONNHAMMER.** *BMC Bioinformatics,* 2005, vol. 6, 298
- **HIGGINS et al.** *Nucleic Acids Res.,* 1994, vol. 22, 4673-4680
- **KABSCH.** *Acta Crystallogr A,* 1978, vol. 34, 827-828
- **DAMM et al.** *Biophysical Journal,* 2006, vol. 90, 4558-4573
- **PETREY ; HONIG.** *Methods in Enzymology,* 2003, vol. 374, 492-509
- **GAUSSIAN.** AMBER. Frisch, Gaussian, Inc,
- QUANTA/CHARMM. Molecular Simulations, Inc,
- Insight II/Discover. Biosym Technologies Inc,
- **GOODFORD.** *J. Med. Chem.,* 1985, vol. 28, 849-857
- **MIRANKER.** *Proteins: Structure, Function, and Genetics,* 1991, vol. 11, 29-34
- **HENDLICH et al.** *Journal of Molecular Graphics and Modeling,* 1997, vol. 15, 359-363
- **EDELSBRUNNER ; KOEHL.** *Proc. Natl. Acad. Sci. USA,* 2003, vol. 100, 2203-2208
- **PROMPANAS et al.** *Bioinformatics,* 2000, vol. 16, 915-922
- **DEL CARPIO et al.** *J. Mol. Graphics,* 1992, vol. 11, 23-29
- **KUNTZ et al.** *J. Mol. Biol.,* 1982, vol. 161, 269-288
- **HO ; MARSHALL.** *J. Comput.-Aided Mol. Design,* 1990, vol. 4, 337-354
- **BARDFORD et al.** *Biochemistry,* 1988, vol. 27, 6733-6741
- **STAHL ; SCHNEIDER.** *Protein Engineering,* 2000, vol. 13, 83-99
- **FETROW ; SKOLNICK.** *Journal of Molecular Biology,* 1998, vol. 281, 949-968
- **WALLACE et al.** *Protein Science,* 1997, vol. 6, 2308-2323
- **LASKOWSKI.** *Journal of Molecular Graphics,* 1995, vol. 13, 323-330
- **WEISEL et al.** *Chemistry Central Journal,* 2007, vol. 1, 7

- **LEVITT.** *J. Mol. Graphics,* 1992, vol. 10, 229-234
- **DELANEY.** *J. Mol. Graphics,* 1992, vol. 10, 174-177
- **LAGUNIN et al.** *Bioinformatics,* 1999, vol. 16, 747-748
- **BRADY ; STOUTEN.** *Journal of Computer-Aided Molecular Design,* 2000, vol. 14, 383
- **LANDGRAF et al.** *Journal of Molecular Biology,* 2001, vol. 307, 1487
- **STUART.** LigBase: a database of families of aligned ligand binding sites in known protein sequences and structures. *Bioinformatics,* 2001, vol. 17, 1-2
- **NEEDLEMAN ; WUNSCH.** *Molecular Biology,* 1970, vol. 48, 443
- **PEARSON ; LIPMAN.** *Proc. Natl. Acad. Sci. USA,* 1988, vol. 85, 2444-2448
- **ALTSCHUL et al.** *Journal of Molecular Biology,* 1990, vol. 215, 403-410
- **ALTSCHUL et al.** *Nucleic Acids Research,* vol. 25, 3389-3402
- **BRUTLAG et al.** *Computational Chemistry,* 1993, vol. 17, 203-207
- **ALTSCHUL et al.** *J. Mol. Biol.,* 1990, vol. 215, 403-410
- **ALTSCHUL et al.** *Methods in Enzymology,* 1996, vol. 266, 460-480
- **KARLIN et al.** *PNAS USA,* 1993, vol. 90, 5873-5787
- **PEARSON.** *Protein Science,* 1995, vol. 4, 1145-1160
- **HIGGIN et al.** *Methods Enzymol.,* 1996, vol. 266, 383-402
- **THOMPSON et al.** *Nucl. Acids Res.,* 2000, vol. 28, 2910-2926
- European Molecular Biology Laboratory. Heidelberg, vol. 1, 69117
- **SALI ; SANCHEZ.** *Methods Mol. Biol.,* 2000, vol. 143, 97-129
- **HENIKOFF ; HENIKOFF.** *Proteins,* 1993, vol. 17, 49-61
- *Pac. Symp. Biocomput.,* 1997, 465-76
- **RISLER et al.** *J. Mol. Biol.,* 1988, vol. 204, 1019-29
- **MYERS ; MILLER.** *CABIOS,* 1988, vol. 4, 11-17
- **HENIKOFF ; HENIKOFF.** *Proc. Natl. Acad. Sci. USA,* 1992, vol. 89, 10915
- **TORELLIS ; ROBOTTI.** *Comput. Appl. Biosci.,* 1994, vol. 10, 3-5
- **TREJO et al.** *J. Med. Chem,* 2003, vol. 46, 4702-4713
- **WESTON ; DAVIS.** *Curr. Opin. Genet. Dev.,* 2002, vol. 12, 14-21